(19) Europäisches Patentamt
European Patent Office
Office européen des brevets

(11) **EP 4 464 313 A2**

(12) **EUROPEAN PATENT APPLICATION**

(43) Date of publication:
**20.11.2024 Bulletin 2024/47**

(21) Application number: **24205501.0**

(22) Date of filing: **25.11.2021**

(51) International Patent Classification (IPC):
**A61K 9/48** *(2006.01)*

(52) Cooperative Patent Classification (CPC):
**A61K 9/4891; A61K 9/4825**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2020 EP 20209913**
**08.02.2021 EP 21155696**

(62) Document number(s) of the earlier application(s) in accordance with Art. 76 EPC:
**21806613.2 / 4 251 126**

(71) Applicant: **Capsugel Belgium NV**
**2880 Bornem (BE)**

(72) Inventors:
• **HE, Xiongwei**
**68000 Colmar (FR)**

• **PALANGETIC, Ljiljana**
**68000 Colmar (FR)**
• **VANQUICKENBORNE, Stefaan**
**2880 Bornem (BE)**
• **CUEVAS, Jerome**
**68000 Colmar (FR)**

(74) Representative: **De Clercq & Partners**
**Edgard Gevaertdreef 10a**
**9830 Sint-Martens-Latem (BE)**

Remarks:
This application was filed on 09.10.2024 as a divisional application to the application mentioned under INID code 62.

(54) **DOUBLE LAYERED DELAYED-RELEASE HARD CAPSULES**

(57) The invention discloses double layered delayed-release hard capsules made by double dipping, that is by two consecutive dip molding steps on the same mold pin without removing the film, provided by the first dip molding step, from the mold pin between the two dip molding steps, but dipping the mold pin with this first film in the second dip molding step, each dip molding step is done with a different polymer melt, providing a double layered delayed-release capsule.

**EP 4 464 313 A2**

**Description**

The invention discloses double layered delayed-release hard capsules made by double dipping, that is by two consecutive dip molding steps on the same mold pin without removing the film, provided by the first dip molding step, from the mold pin between the two dip molding steps, but dipping the mold pin with this first film in the second dip molding step, each dip molding step is done with a different polymer melt, providing a double layered delayed-release capsule.

**BACKGROUND OF THE INVENTION**

Capsules are a common dosage form for pharma, health & nutrition, and are usually required to dissolve in the stomach as fast as possible, releasing their content, but for certain purposes they are designed to pass through the stomach and into the intestine before dissolving. Such capsules are described by a variety of terms, including gastric-resistant, entero-soluble and delayed-release.

GB 2 361 643 A discloses a one piece soft capsule with two layers. Two piece hard capsules are not disclosed. Such one piece soft capsules are not prepared by a mold dipping process but by an encapsulation process which cannot be used for the production of a two piece hard capsule. The core of the invention in said application is that a part of the inner layer is deliberately unshielded by outer layer and hence in contact with the outer environment. Gelatin as first layer or HPMC or HPMCAS are not disclosed.

GB 1,455,884 A discloses capsules which have been prepared by a double dipping process, wherein the first layer is gelatin. HPMC and HPMCAS are not disclosed. The first layer is prepared by a dipping into an aqueous, solvent free melt, the second layer, which can be HPMCP or CAP is prepared by dipping into a solvent based melt.

US 4,816,259 discloses coated soft gelatin capsules. A two piece hard capsule is not disclosed.

US 2013/0295188 A1 discloses a single-layered HPMCAS two piece hard capsule. Two layered capsules are not disclosed.

The US Pharmacopeia Method A describes an in-vitro dissolution test for delayed-release dosage forms such as capsules containing for example a pharmaceutical which calls for a release of the pharmaceutical staying below a certain threshold when treated in a first step with an aqueous medium of pH 1.2, that is 0.1 N HCl, for 2 h, thus simulating the gastric fluid of the stomach, and an increased or better full release when treated in a second step with an aqueous buffer of pH 6.8 for a certain time, thus simulating the intestine.

To achieve both goals is a challenge, either too much of the pharmaceutical is already released in the first step under pH 1.2 conditions, or too little is released in the second step under pH 6.8 conditions.

Target under pH 1.2 conditions as defined by the USP is a release of less than 10% of the content of the capsule after 120 min at pH 1.2.

Target under pH 6.8 conditions as defined by the USP is a release of more than 80% of the content of the capsule after 45 min at pH 6.8.

Next to the desired dissolution requirements, the mechanical properties of the capsules are also important. It is desired to have capsules that are sufficiently stable for storage under different relative humidity conditions. Mechanical properties are for example the capsule's fracture and elasticity behavior (e.g. through the tube test), top strength (e.g. through a capsule top compression test), and (pre)lock force strength (e.g. through a (pre)lock force measurement test) can be important parameters which should be within acceptable ranges. Alternatively for assessing mechanical behavior, several mechanical properties can be measured on films formed by the gelling composition such as: deformation at break (e.g. through a film tensile test), impact resistance (e.g. through an impact energy for fracture test), piercing time by dissolution fluid (e.g. through a piercing time measurement test), or puncture resistance (e.g. through a puncture test).

A balance needs to be found between sufficient enteric characteristics to safeguard enteric release of the active ingredient and suitable mechanical properties of the capsules, also after storage.

In addition to this dissolution test such delayed-release capsules need to fulfill the usual requirements asked for from capsules, such as mechanical properties like resistance against mechanical impact, low brittleness, or high elasticity measured for example by elongation of a polymer film at break. Resistance against mechanical impact may be evaluated by a tube test as shown in Example 5.

Further requirements are low disintegration tendency, integrity of the capsule after 2 h in the aqueous medium of pH 1.2 when the capsule is completely filled or underfilled, the porosity of the capsule, the ability to protect the content from the acid media.

Solvent based melts which contain at least one organic solvent are critical to be handled in production for safety reasons, since specific containment conditions of solvent vapors are required for environmental and health reasons and also measures need to be taken to avoid any ignition or explosion e.g. during production.

In addition there were concerns about residual solvent being present in capsules for e.g. pharmaceutical use when solvent based melts containing organic solvents are used. Due to these concerns of residual solvent, the tendency is to use volatile solvents in non-aqueous melts. The volatility aggravates the mentioned inherent problems linked to the use of organic solvents.

Furthermore, also the dipping and drying properties of a solvent based melt are different from drying properties of aqueous based melts, so any experience in the dipping and drying behavior of solvent based melts can usually not be simply transferred to aqueous melts; it is not foreseeable if what is possible with solvent based melts is also possible with aqueous based melts. Also the behavior and interaction of two layers on the pin when the inner layer is aqueous based and the outer layer is solvent based is different from the situation when both melts are aqueous based. These differences may lead to defects (for example: swellings, wrinkles, bulges or cracks) depending on the various combinations of solvents and polymers in the two layers which again necessitates different process conditions, if such defects are curable at all. Also this different behavior and interaction is not predictable.

A delayed-release capsule prepared by a double dipping process was found to fulfill these requirements.

**Abbreviations and definitions**

| | |
|---|---|
| CAP | cellulose acetate phthalate |
| cP | centipoise, it is one hundredth of a poise, or one millipascal-second (mPa·s) in SI units (1 cP = $10^{-3}$ Pa·s = 1 mPa·s) |
| HPMC | hydroxypropyl methylcellulose, also called hypromellose or Cellulose, 2-hydroxypropyl methyl ether or cellulose hydroxypropyl methyl ether, CAS 9004-65-3. |

The definitions of Hypromellose which are used in instant invention can be found in:

US Pharmacopeia

Official Date: Official as of 1-May-2019

Document Type: USP & NF

DocId: 1_GUID-6 A0B0F3C-FA70-433C-AD55-2020BBC64718_4_en-US

Printed from: https://online.uspnf.com/uspnf/document/1_GUID-6A0B0F3C -FA70-433C-AD55-2020BBC64718_4_en-US

© 2020 USPC

Most Recently Appeared In:
Pharmacopeial Forum: Volume No. 42(5)

| | |
|---|---|
| HPMCAS | hydroxypropyl methylcellulose acetate succinate, hypromellose acetate succinate |
| HPMCP | hydroxypropyl methylcellulose phthalate |
| LOD | Loss on Drying |
| Method A | US Pharmacopeia |

Official Status: Currently Official on 01-Apr-2020

Official Date: Official as of 1-May-2016

Document Type: GENERAL CHAPTER

DocId: 1_GUID-AC788D41-90A2-4F36-A6E7-769954A9ED09_1_en-US

Printed from: https://online.uspnf.com/uspnf/document/1_GUID-AC788D41-90A2-4F36-A6E7-769954A9ED09_1_en-US

© 2020 USPC

General chapter Dissolution (711)

Method A procedure

INTERPRETATION: Delayed-Release Dosage Forms, Acceptance Table 3 and 4

Most Recently Appeared In:
Pharmacopeial Forum: Volume No. 40(6)

| PVA | polyvinyl alcohol |
| STD capsule weight | standard capsule weight, STD capsule weight is dependent on and is predefined for a given capsule size |
| THF | tetrahydrofuran |
| USP | US Pharmacopeia |
| wt% | weight percent, percent by weight |

## SUMMARY OF THE INVENTION

Subject of the invention is a hard capsule shell CAPSSHELL, wherein

CAPSSHELL is prepared by a double dipping process;
the wall of CAPSSHELL comprises two layers of wall forming polymers, one inner layer and one outer layer on the inner layer;
the wall forming polymer of the inner layer comprises a water soluble film forming polymer WATSOLPOL selected from the group of gelatin, cellulose derivative, PVA, and modified starch;
the wall forming polymer of the outer layer comprises a delayed-release polymer ENTPOL selected from the group of HPMCAS, HPMCP, CAP, and polyacrylic acid copolymers.

## BRIEF DESCRIPTION OF THE DRAWINGS

Figure 1: shows a graphical representation of the data of Table 23, Puncture Test: Force at break vs wt% of HPMC in films.

Figure 2: shows a graphical representation of the data of Table 24, Tensile Test: Deformation at break vs wt% of HPMC in films

Figure 3: shows a graphical representation of the data of Table 26, Tensile Test: Elastic modulus vs wt% of gelatin in films

Figure 4: shows a graphical representation of the data of Table 26: Tensile Test: Stress at break vs wt% of gelatin in films

Figure 5: shows a picture of a microtome cut of a double layered film gelatin-HPMCAS. The accolades present the boundaries of both layers, intersected by a partial dashed line. The arrow indicates the infiltration of the blue dye (showing as a darker shade in the black and white picture) through the HPMCAS layer and down to about the middle of the gelatin layer.

Figure 6: shows a picture of a microtome cut of a double layered film HPMC-HPMCAS. The accolades present the boundaries of both layers, intersected by a partial dashed line. The arrow indicates the infiltration of the blue dye (showing as a darker shade in the black and white picture) through the HPMCAS layer and down to about the middle of the HPMC layer.

## DETAILED DESCRIPTION OF THE INVENTION

Double dipping process means that a mold pin is dipped first into a first solution of a first film forming polymer, after the extraction of the mold pin from the first solution the first film forming polymer forms a film on the mold pin, then a second dipping is done of the mold pin, which is covered by the film of the first film forming polymer, into a second solution of a second film forming polymer, which is different from the first film forming polymer, after the extraction of the mold pin from the second solution a film of the second film forming polymer forms on the film of the first film forming polymer which is on the mold pin.

A double dipped capsule comprises two parts, a cap and a body, each the cap and the body are prepared separately by a double dipping process with a respective mold pin shaped to provide either the cap or the body. So a double dipped capsule, once the capsule is filled and closed by fitting the cap onto the body, is distinctly different from a coated capsule, which was not prepared by a double dipping process. Both a coated capsule and a capsule prepared by double dipping have two layers, an inner layer and an outer layer. The coating of the capsule is done with the capsule being in a state, wherein the cap is at least partly engaged telescopically with the body. So the coating is applied onto the capsule with the capsule being in a state, wherein the cap is at least partly engaged telescopically with the body. At least partly engaged means that the cap has been at least partly slid over the body.

Usually two such states are differentiated, a preclosed state and a closed state. In both states the cap engages telescopically with the body, in the preclosed state the cap has been slid over the body only until the preclosed position is reached, so the cap is telescopically only partly engaged with the body. In the closed state the cap is fully engaged with the body.

Usually there are constructive means in the wall of the body and/or the cap that allows fixing the cap in a preclosed state and fixing the cap in the closed state. Such means are usually locking rings, these locking rings are protrusions of the wall of the body and/or the cap which extend at least partly, preferably circumferentially around the body and/or the cap and usually extend radially into the interior of the body and/or the cap. Either the cap or the body may for example have to such protrusion with the respective other part having one such protrusion, for example the body may have one such locking ring and the cap may have two such locking rings separated from each other by a certain distance in axial direction, with the locking ring of the body engaging with the first locking ring of the cap, which is closer to the rim of the open end of the cap, in the preclosed state, and with the second locking ring of the cap, which is closer to the closed end of the cap, in the closed state.

The capsule in the closed state may be called a closed capsule, in the preclosed state a preclosed capsule. Usually the engagement of the cap with the body is a less tight engagement in the preclosed state than in the closed state. So removal of the cap from the body in the preclosed state is still possible without damaging any part of the capsule, whereas separating a cap from a body of a closed capsule is more difficult, part of the capsule may even be damaged.

Usually the capsule is closed only after the capsule has been filled with the desired content, since opening a closed capsule is usually difficult or even impossible without damaging the capsule. Whereas the preclosed position is such that a manageable and defined force, a so called pre-lock force, is required to separate the cap again from the body without damaging cap or body. This separation of the cap from the body of a preclosed capsule is usually done by a machine which subsequently also fills the body with the desired content and then slides the cap over the body until the closed state is reach, thereby closing the capsule. The preclosed capsule is for example used for shipping capsules after manufacture of the capsules but before filling, so cap and body are partly joined and can then be separated again for the filling.

When coating is applied to a capsule in the preclosed state then the capsule usually is empty, whereas when a coating is applied to closed capsule then the coating is applied usually only after the capsule has been filled and closed. The coating covers the outside of the closed or preclosed capsule completely, the slit between the cap and the body, which is visible when an non-coated capsule has been preclosed or closed, and which shows that the cap and the body are indeed two separate parts, is covered by the coating after the coating has been applied onto the closed or preclosed capsule, so the slit is no longer visible or approachable. This is in contrast to a double dipped capsule, where said slit is not covered, but still visible and approachable.

Enteric coating made from polymer dispersions generally show non uniform capsule films due, for example, to particle coalescence issues, whereas in comparison thereto the second layer in case of a double dipped capsule shows good film uniformity. For this reason also the technical performance of double layered capsules shows also less variability, it is less likely to have double dipped capsules with technical performance which is off spec than in case of coated capsules.

Another distinct difference between a coated capsule and a double dipped capsule is the area which is covered by the coating in case of a coated capsule or by a second layer which is applied by a double dipping process: Since a coating is applied to a capsule in a preclosed or closed state from outside, obviously only the area accessibly from the outside will be coated. Since the cap telescopically at least partly engages with the body in case of a preclosed capsule and fully engages with the body in case of a closed capsule, there is an outside area of the body starting from the rim of the open end of the body and extending for a certain distance axially in the direction of the closed end of the body which will not be covered with a coating, since this area is covered by the cap which is telescopically at least partly engaged with the body. So this certain distance is the distance in axial direction from the rim of the open end of the body until the rim of the open end of the cap when the cap is telescopically engaged with the body either in the preclosed or in the closed state.

This area has the form of a ring starting from the rim of the open end of the body, the ring extends circumferentially fully around the body and extends so far from rim of the open end of the body in axially direction towards the closed end of the body as the cap has been slid over the body in the preclosed or closed state, when the coating is applied.

In contrast in case of a second layer that was applied by a double dipping onto the body the complete outside area of the body is covered with the second layer.

The capsule is manufactured by a capsule manufacturing enterprise, whereas the coating in preclosed state with subsequent filling and closing, or the filling, closing and subsequent coating in closed state may be done by a different enterprise or by different enterprises, which may be a tool manufacturer or even a customer of the capsule manufacturer.

CAPSSHELL is a capsule shell for filling with a substance FILL, FILL comprises a substance selected from the group of an active pharmaceutical ingredient, pharmaceutical dosage form, medicament and nutritional product. CAPSSHELL is a capsule of the type used in pharmaceutical or healthcare or nutrition applications.

The term "hard capsule" or "hard capsule shell" refers to a type of capsule or capsule shell that is produced by first producing a capsule shell, and then encapsulating contents in the capsule shell. A hard capsule shell is a two parts or two piece hard capsule shell, the two parts or two pieces are the cap and the body of the capsule shell as further detailed herein.

The wall thickness of CAPSSHELL is known to the skilled person, a typical value may be around 100 micrometer, a typical range may be from 60 to 150 micrometer.

Typical sizes of CAPSSHELL are known to the skilled person and may be for example expressed in the sizes 000, 00el, 00, 0el, 0, 1, 2, 3, 4, 5, or 9, for example as disclosed in Pharmaceutical Capsules, Second Edition, 2014, edited by F. Podczeck and B. E. Jones, Pharmaceutical Press, London, UK, page 84, Table 4.1. Closed joined length of CAPSSHELL may vary from 11 to 27 mm, cap length from 6 to 13 mm, body length from 9 to 23 mm, cap diameter from 4 to 10 mm and body diameter from 4 to 10 mm.

CAPSSHELL comprises two halves of a capsule shell, the two halves are called the cap and the body of the capsule shell.

The term "half" does not mean that cap and body are of equal size; rather the term "half" is to be understood as "part" or "piece", so CAPSSHELL comprises the two parts or the two pieces of a capsule, one part which is the cap, and the another part which is the body.

The cap and the body are two separate parts. When joined together they form the capsule, or the capsule shell, which can be empty or filled. The words "capsule" and "capsule shell" are usually used interchangeably. The term "shell" refers usually to the capsule shaped polymer which forms the film which again forms the wall of the shell, that again is the shell, so the capsule shaped polymer is also called the shell.

The cap and the body may be telescopically engageable to provide CAPSSHELL. Typically, the cap and the body have each two regions, a dome shaped region, which is the closed end of the cap or of the body respectively, and an essentially cylindrically shaped region, which extends from the dome shaped region and which ends with the open end of the cap or of the body respectively.

The essentially cylindrically shaped region of the cap, or at least part of it, is telescopically engageable with the essentially cylindrically shaped region of the body, or at least with part of it. It is essentially an inserting of the body into the cap or vice versa. This inserting is typically a sliding of the cap over the body or vice versa. Typically the cap slides over the body. Thereby the essentially cylindrically shaped region of the body, or at least part of this region of the body, is inside the cavity of the essentially cylindrically shaped region of the cap, or at least inside a part of the cavity of this region of the cap. So the essentially cylindrically regions, or part of them, of cap and body slide over the other one, as the case may be. So typically the body is inserted into the cap, i.e. the body slides into the cap. The telescopical engagement happens co-axial with respect to the longitudinal axis of the cap and the body. So the telescopically engaged cap and body is the capsule.

CASPSSHELL in the sense of this invention means the cap, the body, or both, such as both engaged with each other.

The longitudinal or axial direction is the direction from the closed end of the cap to the closed end of the body and vice versa, whereas the radial direction is perpendicular to the longitudinal direction.

The terms "inner" and "outer" and the terms "inside" and "outside" refer to the cavity, which is the inside of the body or the cap or of CAPSSHELL respectively, and to the exterior of the body or the cap or of CAPSSHELL respectively. So for example when the outer layer is defined to be on the inner layer then this means that the inner layer is facing to the cavity whereas the outer layer is facing to the exterior of CAPSSHELL.

CAPSSHELL is prepared by a double dipping process. This means that the cap is prepared by a respective double dipping process and the body is prepared by a respective double dipping process.

A double dipping process as disclosed herein, also with all its embodiments, may be used for the preparation of CAPSSHELL.

CAPSSHELL is not a coated capsule.

CAPSSHELL is not prepared by a coating process wherein the coating is applied to CAPSSHELL with CAPSSHELL being in a closed or preclosed state during the application of the outer layer; in particular the outer layer of wall forming polymer of CAPSSHELL is not applied onto the inner layer of wall forming polymer of CAPSSHELL by a coating process, wherein the outer layer is applied onto the inner layer with CAPSSHELL being in a closed or preclosed state during the application of the outer layer.

CAPSSHELL is not prepared by a coating process; in particular the outer layer of wall forming polymer of CAPSSHELL is not applied onto the inner layer of wall forming polymer of CAPSSHELL by a coating process.

So the outer layer of CAPSSHELL is not applied onto the inner layer of CAPSSHELL by a coating process with CAPSSHELL being in a closed or preclosed state during the application of the outer layer.

The outside of the inner layer of the body of CAPSSHELL is completely covered by the outer layer of the body. Completely covered means that the outer layer covers the outside of inner layer the body including the closed end of the body and from the closed end of the body up to the rim of the open end of the body, this covering of the outside of the inner layer of the body by the outer layer of the body is without any gap and without any area of the outside of the inner layer of the body not being covered by the outer layer. Especially no circumferential area of the inner layer around the body starting from the rim of the open end of the body and extending axially towards the closed end of the body for a certain distance is not covered by the outer layer of the body.

The inner layer of the body of CAPSSHELL does not have an area on its outside which is not covered by the outer layer; specifically the outside of the inner layer of the body does not have an area extending circumferentially around the body and staring from the rim of the open end of the body and extending longitudinally for a certain distance in the direction of the closed end of the body.

Preferably, WATSOLPOL is gelatin or cellulose derivative.

Cellulose derivative include water-soluble cellulose ethers in which one or more hydrogen atoms of hydroxy groups of cellulose are substituted with at least one substituent selected from the group of alkyl groups, such as $C_{1-4}$ alkyl groups, and hydroxyalkyl groups, such as $C_{1-4}$ hydroxyalkyl groups. Specific examples include hydroxy lower alkyl celluloses such as methyl cellulose, hydroxypropylcellulose (HPC) and hydroxyl-lower alkylalkyl celluloses such as hydroxyethylmethylcellulose, hydroxyethylethylcellulose, and hydroxypropylmethylcellulose (HPMC). Among them, hydroxypropylmethylcellulose (HPMC) is particularly preferred.

More preferably, WATSOLPOL is gelatin or HPMC.

Suitable WATSOLPOL are commercially available.

The HPMC may have a methoxy content of 27.0 to 30.0 % (w/w).

The HPMC may have a hydroxypropoxy content of 4.0 to 12.0 % (w/w).

Preferably, the HPMC may have a methoxy content of 27.0- to 30.0 % (w/w) and a hydroxypropoxy content of 4.0 to 12.0 % (w/w).

In the instant invention the HPMC methoxy and hydroxypropoxy contents are expressed according to US Pharmacopeia as the US Pharmacopiea reference is cited herein.

There are different types or grades of HPMC.

Therefore the term HPMC in the sense of the invention also comprises mixtures of more than one type or grades of HPMC.

The HPMC may for example be selected from the group of

HPMC 2910 containing about 7.0 to 12.0% hydroxypropoxy group and about 28.0 to 30.0% methoxy group,
HPMC 2906 containing about 4.0 to 7.5% hydroxypropoxy group and about 27.0 to 30.0% methoxy group,
HPMC 2208 containing about 4.0 to 12.0% of hydroxypropoxy group and about 19.0 to 24.0% of methoxy group,
HPMC 1828 containing about 23.0 to 32.0% hydroxypropy group and about 16.5 to 20.0% methoxy group, and

mixtures thereof.

The HPMC in CAPSSHELL may be one type of HPMC, it may also be a mixture of different types of HPMC.

In one embodiment, the HPMC is HPMC 2906.

The wall forming polymer of the inner layer may comprise a mixture of more than one WATSOLPOL.

Preferably, ENTPOL is selected from the group of HPMCAS or CAP.

In one embodiment, ENTPOL is HPMCAS.

The wall forming polymer of the inner layer may comprise a mixture of more than one ENTPOL.

In one embodiment, WATSOLPOL is cellulose derivative, preferably HPMC, and ENTPOL is HPMCAS.

In another embodiment, WATSOLPOL is gelatin and ENTPOL is HPMCAS or CAP.

In another embodiment, WATSOLPOL is gelatin and ENTPOL is HPMCAS.

In another embodiment, WATSOLPOL is gelatin and ENTPOL is CAP.

Based on the STD capsule weight;

the amount of ENTPOL in CAPSSHELL may be at least 5 wt%, preferably at least 7.5 wt%, more preferably at least 10 wt%, even more preferably at least 12.5 wt%, especially at least 15 wt%, more especially at least 17.5 wt%, even more especially at least 18.5 wt%, in particular at least 20 wt%, the wt% being based on STD capsule weight.
The amount of ENTPOL in CAPSSHELL may be up to 50 wt%, preferably up to 45 wt%, more preferably up to 40 wt%, even more preferably up to 35 wt%, the wt% being based on STD capsule weight.
Any of the minimum amount of ENTPOL may be combined with any of the maximum amount of ENTPOL.

For example, the amount of ENTPOL in CAPSSHELL may be from 5 to 50 wt%, preferably from 7.5 to 45 wt%, more preferably from 9 to 40 wt%, even more preferably from 10 to 35 wt%, the wt% being based on STD capsule weight.

Based on the dry capsule weight;

the amount of ENTPOL in CAPSSHELL may be at least 5 wt%, preferably at least 7.5 wt%, more preferably at least 9.5 wt%, the wt% being based on dry capsule weight.

The amount of ENTPOL in CAPSSHELL may be up to 50 wt%, preferably up to 45 wt%, more preferably up to 40 wt%, even more preferably up to 35 wt%, especially up to 32.5 wt%, the wt% being based on dry capsule weight.

Any of the minimum amount of ENTPOL may be combined with any of the maximum amount of ENTPOL.

For example, the amount of ENTPOL in CAPSSHELL may be from 5 to 50 wt%, preferably from 7.5 to 45 wt%, more preferably from 7.5 to 40 wt%, even more preferably from 7.5 to 35 wt%, especially from 9.5 to 35 wt%, more especially from 9.5 to 32.5 wt%, the wt% being based on dry capsule weight.

In case that ENTPOL is HPMCAS, the amount of HPMCAS in CAPSSHELL may be from 12 to 32.5 wt%, preferably from 12 to 31 wt%, the wt% being based on dry capsule weight.

In case that ENTPOL is HPMCAS-LG, the amount of HPMCAS-LG in CAPSSHELL may be from 13.5 to 32.5 wt%, preferably from 13.5 to 31 wt%, the wt% being based on dry capsule weight.

In case that ENTPOL is HPMCAS-HG, the amount of HPMCAS-HG in CAPSSHELL may be from 11.5 to 22.5 wt%, the wt% being based on dry capsule weight.

In case that ENTPOL is CAP, the amount of CAP in CAPSSHELL may be from 9.5 to 22.5 wt%, the wt% being based on dry capsule weight.

Based on the STD capsule weight;

the amount of WATSOLPOL in CAPSSHELL may be up to 95 wt%, preferably up to 92.5 wt%, more preferably up to 90 wt%, even more preferably up to 87.5 wt%, especially up to 85 wt%, more especially up to 82.5 wt%, even more especially up to 81.5 wt%, in particular up to 80 wt%, the wt% being based on STD capsule weight.
The amount of WATSOLPOL in CAPSSHELL may be at least 50 wt%, preferably at least 55 wt%, more preferably at least 60 wt%, even more preferably at least 65 wt%, the wt% being based on STD capsule weight.
Any of the minimum amount of WATSOLPOL may be combined with any of the maximum amount of WATSOLPOL.
For example, the amount of WATSOLPOL in CAPSSHELL may be from 50 to 95 wt%, preferably from 55 to 92.5 wt%, more preferably from 60 to 90 wt%, even more preferably from 65 to 87.5 wt%, the wt% being based on STD capsule weight.

Based on the dry capsule weight;

the amount of WATSOLPOL in CAPSSHELL may be up to 95 wt%, preferably up to 92.5 wt%, more preferably up to 90 wt%, the wt% being based on dry capsule weight.
The amount of WATSOLPOL in CAPSSHELL may be at least 50 wt%, preferably at least 55 wt%, more preferably at least 60 wt%, even more preferably at least 65 wt%, especially at least 67.5 wt%, the wt% being based on dry capsule weight.
Any of the minimum amount of WATSOLPOL may be combined with any of the maximum amount of WATSOLPOL.
For example, the amount of WATSOLPOL in CAPSSHELL may be from 50 to 95 wt%, preferably from 55 to 92.5 wt%, more preferably from 60 to 90 wt%, even more preferably from 65 to 90 wt%, especially 67.5 to 90 wt%, the wt% being based on dry capsule weight.
In case that WATSOLPOL is HPMC, the amount of HPMC in CAPSSHELL may be from 67.5 to 88 wt%, preferably from 67.5 to 86.5 wt%, more preferably from 67.5 to 85 wt%, the wt% being based on dry capsule weight.
In case that WATSOLPOL is gelatin, the amount of gelatin in CAPSSHELL may be from 67.5 to 90 wt%, preferably from 67.5 to 86.5 wt%, in another embodiment from 77.5 to 90.5 wt%, preferably from 77.5 to 88.5 wt%, the wt% being based on dry capsule weight.

In one embodiment,

the amount of WATSOLPOL in CAPSSHELL may be from 67.5 to 90.5 wt%, preferably from 67.5 to 86.5 wt%, in another embodiment from 77.5 to 90.5 wt%, preferably from 77.5 to 88.5 wt%,
and
the amount of ENTPOL in CAPSSHELL may be from 9.5 to 32.5 wt%, preferably from 13.5 to 32.5 wt%, in another embodiment from 9.5 to 22.5 wt%, preferably from 11.5 to 22.5 wt%;
the wt% being based on dry capsule weight.

In case that WATSOLPOL is HPMC and ENTPOL is HPMCAS,

the amount of HPMC in CAPSSHELL may be from 67.5 to 90 wt%, and
the amount of HPMCAS in CAPSSHELL may be from 10 to 32.5 wt%; preferably,
the amount of HPMC in CAPSSHELL may be from 67.5 to 88 wt%, and
the amount of HPMCAS in CAPSSHELL may be from 12 to 32.5 wt%; the wt% being based on dry capsule weight.

In case that WATSOLPOL is HPMC and ENTPOL is HPMCAS-HG,

the amount of HPMC in CAPSSHELL may be from 67.5 to 90 wt%, and
the amount of HPMCAS-LG in CAPSSHELL may be from 10 to 32.5 wt%; preferably,
the amount of HPMC in CAPSSHELL may be from 75 to 90 wt%, and
the amount of HPMCAS-LG in CAPSSHELL may be from 10 to 25 wt%; more preferably,
the amount of HPMC in CAPSSHELL may be from 80 to 90 wt%, and
the amount of HPMCAS-LG in CAPSSHELL may be from 10 to 20 wt%; even more preferably,
the amount of HPMC in CAPSSHELL may be from 82 to 88 wt%, and
the amount of HPMCAS-LG in CAPSSHELL may be from 12 to 18 wt%; the wt% being based on dry capsule weight.

In case that WATSOLPOL is HPMC and ENTPOL is HPMCAS-LG,

the amount of HPMC in CAPSSHELL may be from 67.5 to 86.5 wt%, and
the amount of HPMCAS-LG in CAPSSHELL may be from 13.5 to 32.5 wt%, the wt% being based on dry capsule weight.

In case that WATSOLPOL is gelatin and ENTPOL is HPMCAS-LG,

the amount of gelatin in CAPSSHELL may be from 67.5 to 86.5 wt%, and
the amount of HPMCAS-LG in CAPSSHELL may be from 13.5 to 32.5 wt%, the wt% being based on dry capsule weight.

In case that WATSOLPOL is gelatin and ENTPOL is HPMCAS-HG,

the amount of gelatin in CAPSSHELL may be from 77.5 to 88.5 wt%, and
the amount of HPMCAS-HG in CAPSSHELL may be from 11.5 to 22.5 wt%, the wt% being based on dry capsule weight.

In case that WATSOLPOL is gelatin and ENTPOL is CAP,

the amount of gelatin in CAPSSHELL may be from 77.5 to 90.5 wt%, and
the amount of CAP in CAPSSHELL may be from 9.5 to 22.5 wt%,

the wt% being based on dry capsule weight.
Based on the STD capsule weight;
the amount in wt% of WATSOLPOL in CAPSSHELL is at most [100 wt% - amount of ENTPOL in wt%], with the wt% being based on the STD capsule weight, for the case that CAPSHELL contains only WATSOLPOL and ENTPOL but no further substances.
Any of the amounts and their ranges in wt% of WATSOLPOL in CAPSSHELL may be combined with any of the amounts and their ranges in wt% of ENTPOL in CAPSSHELL, while the combined amounts of WATSOLPOL and of ENTPOL shall not exceed 100 wt%, with the wt% being based on the STD capsule weight.

Based on the dry capsule weight;
the amount in wt% of WATSOLPOL in CAPSSHELL is at most [100 wt% - amount of ENTPOL in wt%], with the wt% being based on the dry capsule weight, for the case that CAPSHELL contains only WATSOLPOL and ENTPOL, but no further substances.

Any of the amounts and their ranges in wt% of WATSOLPOL in CAPSSHELL may be combined with any of the amounts and their ranges in wt% of ENTPOL in CAPSSHELL, while the combined amounts of WATSOLPOL and of ENTPOL shall not exceed 100 wt%, with the wt% being based on the dry capsule weight.

CAPSSHELL may comprise water. The water may stem from the production process which may be using an aqueous composition for preparing CAPSSHELL, so the water in CAPSSHELL is typically residual water remaining in CAPSSHELL after drying, and it may stem from the humidity of the atmosphere, essentially of the relative humidity of the air, surrounding CAPSSHELL. Typical upper limit of the amount of residual water in CAPSSHELL is 20 wt% or less, preferably 17.5 wt% or less, the wt% being based on the weight of the CAPSSHELL.

Typical lower limit of the amount of residual water in CAPSSHELL is 3 wt% or more, preferably 4 wt% or more, more preferably 5 wt% or more, the wt% being based on the weight of the CAPSSHELL.

Any of the lower limit of the amount of residual water may be combined with any of the upper limit of the amount of residual water.

For example, the amount of residual water in CAPSSHELL may be from 3 to 20 wt%, preferably from 4 to 17.5 wt%, more preferably from 5 to 17.5 wt%, the wt% being based on the weight of the CAPSSHELL.

The amount of residual water may be characterized by the LOD. The dry capsule weight is the capsule weight minus the weight that is lost when the LOD is determined, that is the remaining capsule weight after the LOD determination.

The inner or the outer layer or both layers of CAPSSHELL may further comprise a gelling system GELSYS.

GELSYS may be one or more gelling agents GELAGE or a mixture of one or more GELAGE with one or more gelling aids GELAID.

Typical GELAGE is selected from the group of hydrocolloid such as agar gum, guar gum, locust bean gum (carob), carrageenan, pectin, xanthan, gellan gum, konjac mannan, gelatin, and mixtures thereof;
preferably, GELAGE is selected from the group of carrageenan, pectin, gellan gum, gelatin, and mixtures thereof.

Typical GELAID is a cation; the cation may be $K^+$, $Na^+$, $Li^+$, $NH_4^+$, $Ca^{2+}$, $Mg^{2+}$ or mixtures thereof.

GELSYS may be contained in the inner layer, in the outer layer or in both. CAPSSHELL may comprise from 0.01 to 10 wt%, preferably from 0.01 to 5 wt%, more preferably from 0.01 to 2 wt%, even more preferably from 0.01 to 1 wt%, of GELSYS, the wt% being based on the weight of WATSOLPOL, in the case that GELSYS is contained in the inner layer, or being based on the weight of ENTPOL, in the case that GELSYS is contained in the outer layer.

In one embodiment, the inner layer does not comprise any GELSYS besides gelatin, and WATSOLPOL is gelatin.

In one embodiment, the inner layer does not comprise any GELSYS, and WATSOLPOL is a cellulose derivative, preferably HPMC.

In one embodiment, the outer layer does not comprise any GELSYS.

In one embodiment, the inner layer does not comprise any GELSYS besides gelatin, and WATSOLPOL is gelatin, and the outer layer does not comprise any GELSYS.

In one embodiment, the inner layer does not comprise any GELSYS, and WATSOLPOL is a cellulose derivative, preferably HPMC, and the outer layer does not comprise any GELSYS.

CAPSSHELL may comprise one or more further substances FURTHERSUBST, FURTHERSUBST is selected from the group of plasticizer, pH regulator, sweetener, acidulant, preservative, flavor, binder, thickener, colorant, and mixtures thereof.

FURTHERSUBT may be contained in the inner layer, in the outer layer or in both. Possible content of FURTHERSUBST in each of the layers may be from 0.1 to 20 wt%, preferably from 1 to 10 wt%; the wt% may be based on the weight of WATSOLPOL, in the case that FURTHERSUBST is contained in the inner layer, or may be based on the weight of ENTPOL, in the case that FURTHERSUBST is contained in the outer layer.

Examples of the plasticizer include triethyl citrate, glycerin, D sorbitol (D-sorbitol/sorbitan solution), D-mannitol, trehalose, a vegetable oil (sesame oil, castor oil), a medium chain triglyceride, a triacetin, a phthalate (dioctyl phthalate), a phytosterol, a propylene glycol, polysorbate and a polyethylene glycol (macro goal).

Examples of the pH regulator include phosphoric acid, hydrochloric acid, citric acid, glycine, gluconic acid, succinic acid, acetic acid, tartaric acid, lactic acid, fumaric acid, boric acid, maleic acid, sulfuric acid, malic acid, ammonia, a hydroxide, an amine, and salts thereof.

Examples of the sweetener include aspartame, acesulfame potassium, amacha powder, liquid sugar, fructose, glucose, reduced maltitol syrup, licorice, xylitol, glycine, glycerin, glycyrrhizinate, brown sugar, saccharin, sucralose, stevia extract, refined white sugar, purification honey, D-sorbitol, maltitol, maltose and D-mannitol.

Examples of the acidulant include adipic acid, itaconic acid, citric acid, trisodium citrate, glucono-delta-lactone, gluconic acid, potassium gluconate, sodium gluconate, succinic acid, monosodium succinate, disodium succinate, sodium acetate, tartaric acid, lactic acid, sodium lactate, acetic acid, phytic acid, fumaric acid, malic acid and phosphoric acid.

Examples of the preservative include benzoic acid, sodium benzoate, p-hydroxybenzoate, sodium sulfite, sodium hyposulfite, sodium pyrosulfite, potassium pyrosulfite, propionic acid, calcium propionate, sodium propionate, storax extract, capillary artemisia extract, Milt protein extract, a sorbic acid compound, sodium dehydroacetate, nysin, sulfur dioxide, a pectin degradation product and epsilon-polylysine.

Examples of the flavor include various essences, flavors, peppermint, menthol, cinnamon, fennel, vanilla, lemon, and camphor.

The sweetener, acidulant and flavor are suitably used when FILMCOMP is used for edible products.

Examples of the thickener include alginic acid, alginate, gum Arabic, karaya gum, guar gum, gellan gum, tamarind seed gum, tara gum, tragacanth gum, carrageenan, CMC-Ca, CMC-Na, glucosamine, pullulan, pectin, sodium polyacrylate, methylcellulose, curdlan and modified starch.

The strength of a capsule film can be increased by use of the thickener and binder.

A colorant may be a dye or a pigment with a black, white, such as $TiO_2$, grey or any chromatic color. The term chromatic color herein refers to all colors except black, white and grey.

In one embodiment, when WATSOLPOL is HPMC and ENTOL is polyacrylic acid copolymer, the inner layer does not contain sodium lauryl sulfate, Tween-80, glycerin or polyethylene glycol.

In one embodiment, when ENTPOL is HPMCP, then WATSOLPOL does not contain both HPMC and pectin.

In one embodiment CAPSHELL does not have an enteric coating on the inside of the inner layer.

A known process for the production of capsules is dip molding, a mold pin is dipped into a so called melt, which can also be called bath, which is a mixture of a film forming polymer with a solvent, usually a solution of the film forming polymer in the solvent, and subsequent extraction of the mold pin from the melt, thereby the polymer forms a film on the mold pin after drying.

The cap may be obtained from by a mold pin having the respective geometric shape complementary to the desired shape of the cap. The body may be formed by a mold pin having the respective geometric shape complementary to the desired shape of the body. By using the respective mold pin in the dip molding either the cap or the body is obtained.

Further subject of the invention is a method for preparation of CAPSSHELL by a dip molding process DOUBLEDIP,

in DOUBLEDIP a first dip molding DIP1 and a second dip molding DIP2 are done consecutively;
in DIP1 a first polymer film of WATSOLPOL is formed on a mold pin by dipping the mold pin into a first mixture MELT 1 and withdrawing it from the MELT 1,
MELT1 comprises WATSOLPOL and water;
in DIP2 a second polymer film of ENTPOL is formed on the first polymer film on the mold pin by dipping the mold pin with the first polymer film from DIP1 into a second mixture MELT2 and withdrawing it from the MELT1,
MELT2 comprises ENTPOL and water;
with CAPSSHELL, WATSOLPOL and ENTPOL as defined herein, also with all their embodiments.

DOUBLEDIP may also be called a double dipping process.

The first polymer film of WATSOLPOL forms on a mold pin after DIP1 by drying WATSOLPOL on the mold pin.

The second polymer film of ENTPOL forms on the first polymer film on the mold pin by drying ENTPOL on the mold pin, that is by drying ENTPOL on the first polymer film on the mold pin.

Any drying of the first polymer film and of the second polymer film may be done by air drying.

Usually, MELT1 does not comprise ENTPOL.

Usually, MELT2 does not comprise WATSOLPOL except for gelatin, which may be comprised in MELT2, for example as gelling agent.

Preferably, MELT2 does not comprise WATSOLPOL.

In one embodiment, MELT1 does not comprise ENTPOL and MELT2 does not comprise WATSOLPOL except for gelatin, which may be comprised in MELT2, for example as gelling agent.

Preferably, MELT1 does not comprise ENTPOL and MELT2 does not comprise WATSOLPOL.

The mixtures MELT1 and MELT2 are also called melts by the skilled person.

MELT1 and MELT2 need to be provided for DOUBLEDIP.

MELT1 is preferably a solution of WATSOLPOL in water.

MELT2 is preferably a solution of ENTPOL in water.

In one embodiment MELT1 is an aqueous based melt.

In one embodiment MELT2 is an aqueous based melt.

Preferably, MELT1 and MELT2 are aqueous based melts.

In one embodiment MELT 1 is not a solvent based melt.

In one embodiment MELT2 is not a solvent based melt.

Preferably, MELT1 and MELT2 are not solvent based melts.

In one embodiment no further solvent besides water is present in MELT1.

In one embodiment no further solvent besides water is present in MELT2.

In one embodiment no further solvent besides water is present in MELT1 and in MELT2.

ENTPOL is a polymer having acidic residues.

MELT2 further comprises a base BAS2, BAS2 is ammonia.

Ammonia may also be referred to or used as ammonium hydroxide.

BAS2 is added to MELT2 to solubilize ENTPOL in MELT2.

Preferably, MELT2 comprises BAS2 in at least such an amount that ENTPOL is dissolved in MELT2.

The amount of BAS2 in MELT2 may be at least 1 molar equivalent, preferably at least 1.05 molar equivalents, more preferably at least 1.1 molar equivalents, the molar equivalents being based on the molar amount of acidic residues in ENTPOL.

The amount of BAS2 in MELT2 may be up to 1.5 molar equivalents, preferably up to 1.4 molar equivalents, the molar equivalents being based on the molar amount of acidic residues in ENTPOL.

Any of the lower limits of the amounts of BAS2 may be combined with any of the upper limits of the amounts of BAS2.

Preferably, the amount of BAS2 in MELT2 may be from 1 to 1.5 molar equivalents, preferably from 1.05 to 1.4 molar equivalents, more preferably from 1.1 to 1.4 molar equivalents, the molar equivalents being based on the molar amount of acidic residues in ENTPOL.

MELT2 may be prepared by mixing SOL2 and ENTPOL and BAS2.

DIP1 comprises the steps of

(1-1) dipping the mold pin for one of the halves of CAPSSHELL into MELT1;
(1-2) withdrawing the mold pin from MELT1;
(1-3) allowing the first polymer film to form on the mold pin by drying.

DIP2 comprises the steps of

(2-1) dipping the mold pin with the first polymer film from DIP1 into MELT2;
(2-2) withdrawing the mold pin from MELT2;
(2-3) allowing the second polymer film to form on the first polymer film on the mold pin by drying.

The first polymer film is the inner layer of CAPSSHELL.

The second polymer film is the outer layer of CAPSSHELL.

The time between DIP1 and DIP2 usually is short, such as seconds, minutes or at most few hours. In production the double dipping process is done in an automated and continuous way by a respective machine, DIP2 is usually done within a short period of time after the first dip, such as some seconds or some minutes, at most an hour after DIP1.

In one embodiment, a time DRYTIME1-2 is the time between the withdrawal of the mold pin from the MELT1 in DIP1 and the dipping of the mold pin into MELT2, that is the total time between the first and the second dipping. During DRYTIME1-2 the first polymer film dries on the mold pin before the second dip. Preferably DRYTIME1-2 is not longer than 5 h, more preferably not longer than 2 h, even more preferably not longer than 1.5 h, especially not longer than 1 h.

DRYTIME 1-2 may be from 1 sec to 5 h, preferably from 2 sec to 2 h, more preferably from 5 sec to 1.5 h, even more preferably from 5 sec to 1 h.

DIP2 provides a half of CAPSSHELL.

After DIP2 this half of CAPSSHELL is removed from the mold pin.

After DIP2 and before removal of the half of CAPSSHELL from the mold pin the half of CAPSSHELL on the mold pin may be further dried on the mold pin.

Also after removal of the half of CAPSSHELL from the mold pin or after any further steps such as dimensioning such as cutting or assembling the cap and the body, CAPSSHELL may be further dried. Also a additional post-treatment may be applied for example to reduce any residual ammonia in CAPSSHELL to a concentration under a certain predefined maximum residual ammonia concentration in CAPSSHELL.

DOUBLEDIP is done preferably both with a mold pin which is shaped to provide for the cap, which provides this one half of CAPSSHELL, and with a mold pin which is shaped to provide the body, which provides the other half of CAPPSSHELL.

After the preparation of both halves of CAPSSHELL, cap and body, are joined with each other to form the capsule.

The half which is removed from the mold pin may have a length which is still longer than the target length of the desired final half of a capsule shell, in this case the half on the mold pin and after removal from the mold pin represents an unmachined part, and is cut to the desired length to provide the desired final half of a capsule shell in the desired length.

In order to form uniform thickness of the inner layer shell on the pin by dip molding, MELT1 needs to have gelling ability. The gelling nature of a melt is called conventional gelling if the melt is liquid above its gelling temperature and gels below its gelling temperature. Gelatin for example shows the gelling nature conventional gelling.

Whereas the gelling nature, where the melt is liquid below its gelling temperature and gels at temperatures above its gelling temperature, is called thermal gelling. HPMC for example shows the gelling nature thermal gelling.

Depending on the gelling nature of MELT 1, the mold pin may have a higher or a lower temperature PINTEMPDIP1 with respect to the temperature of MELT1 in DIP1,

In one embodiment, PINTEMPDIP1 is above the temperature of MELT1 in DIP1, such as in case of thermal gelling, for example in case of HPMC.

In another embodiment, PINTEMPDIP1 is below the temperature of MELT1 in DIP1, such as in case of conventional gelling, for example in case of gelatin.

The gelling temperature of MELT1 may be determined by a measurement of the viscosity by progressively heating or cooling the solution. The temperature at which the viscosity starts to sharply increase is considered as the gelling temperature. As an example, for a concentration of about 20 wt% in water, HPMC type 2906 has a gelling temperature of about between 30 and 40 °C.

PINTEMPDIP1 may be 5 °C or more, preferably 10 °C or more, more preferably 15 °C or more, above the gelling temperature of MELT 1 in case of thermal gelling.

PINTEMPDIP1 may be 5 °C or more, preferably 10 °C or more, more preferably 15 °C or more, below the gelling temperature of MELT 1 in case of conventional gelling.

Any drying of the polymer film on the mold pin after DIP1 or after DIP2 may be done by air drying.

Embodiments of process claims for preparing a capsule with a combination of an inner layer of HPMC and an outer layer of HPMCAS by double dipping, first in a HPMC melt, that is MELT1 being a HPMC melt, and then in a HPMCAS melt, that is MELT2 being a HPMCAS melt, this combination is also called HPMC-HPMCAS combination in the following, are:

The viscosity of MELT 1, in case that the first polymer is HPMC and the second polymer is HPMCAS, that is the HPMC-HPMCAS combination, may be from 500 to 2500 cP, preferably from 800 to 1000 cP at the temperature of MELT1.

The temperature of MELT 1, in case that the first polymer is HPMC and the second polymer is HPMCAS, that is the HPMC-HPMCAS combination, may be from 25 to 40 °C, preferably from 31 to 33 °C.

PINTEMPDIP1, in case that the first polymer is HPMC and the second polymer is HPMCAS, that is the HPMC-HPMCAS combination, may be from 50 to 80 °C, preferably from 66 to 70 °C.

In preferred embodiments the following parameters apply to the HPMC-HPMCAS combination, i.e. in case that the first polymer is HPMC and the second polymer is HPMCAS:

H1-1) The viscosity of MELT1 may be from 500 to 2500 cP, preferably from 800 to 1000 cP at the temperature of MELT 1, and
the temperature of MELT1 may be from 25 to 40 °C, preferably from 31 to 33 °C; or
H1-2) the viscosity of MELT1 may be from 500 to 2500 cP, preferably from 800 to 1000 cP at the temperature of MELT 1, and
PINTEMPDIP1 may be from 50 to 80 °C, preferably from 66 to 70 °C; or
H1-3) the temperature of MELT1 may be from 25 to 40 °C, preferably from 31 to 33 °C, and
PINTEMPDIP1 may be from 50 to 80 °C, preferably from 66 to 70 °C; or
H1-4) the viscosity of MELT1 may be from 500 to 2500 cP, preferably from 800 to 1000 cP at the temperature of MELT 1,

the temperature of MELT1 may be from 25 to 40 °C, preferably from 31 to 33 °C, and
PINTEMPDIP1 may be from 50 to 80 °C, preferably from 66 to 70 °C.

In any one of the embodiments regarding the process claims of the HPMC-HPMCAS combination above, the drying of the first polymer film on the mold pin, in case that the first polymer is HPMC and the second polymer is HPMCAS, may be done in one stage, DRY1, or in two consecutive stages, a first drying, DRY1-1, and thereafter a second drying, DRY1-2, with DRY1-1 and DRY1-2 having different drying parameters.

DRY1 may be done at a temperature of from 30 to 80 °C, preferably from 40 to 65 °C.

DRY1 may be done at a RH of from 20 to 60 %, preferably from 30 to 50 %.

The time of DRY1-1 may be from 1 to 60 min, preferably from 2 to 55 min, more preferably from 20 to 55 min.

DRY1-1 may be done at a temperature of from 40 to 80 °C, preferably from 50 to 65 °C.

DRY1-1 may be done at a RH of from 30 to 60 %, preferably from 40 to 50 %.

The time of DRY1-1 may be from 1 to 10 min, preferably from 2 to 8 min.

DRY1-2 may be done at a temperature of from 30 to 60 °C, preferably from 40 to 50 °C.

DRY1-2 may be done at a RH of from 20 to 50%, preferably from 30 to 40 %.

The time of DRY1-2 may be from 20 to 60 min, preferably from 30 to 55 min.

In any one of the embodiments regarding the process claims of the HPMC-HPMCAS combination above, that is in case that the first polymer is HPMC and the second polymer is HPMCAS, preferred embodiments for the drying in one stage, DRY1, or in two consecutive stages, a first drying, DRY1-1, and thereafter a second drying, DRY1-2, of the first polymer film on the mold pin, are:

H11-a) DRY1 may be done at a temperature of from 30 to 80 °C, preferably from 40 to 65 °C, and DRY1 may be done at a RH of from 20 to 60 %, preferably from 30 to 50 %; or

H11-b) the time of DRY1-1 may be from 1 to 60 min, preferably from 2 to 55 min, more preferably from 20 to 55 min; or

H11-ab) a combination of H11-a) with H11-b); and

H1-a) DRY1-1 may be done at a temperature of from 40 to 80°C, preferably from 50 to 65 °C, and DRY1-1 may be done at a RH of from 30 to 60 %, preferably from 40 to 50 %; or

H1-b) DRY1-2 may be done at a temperature of from 30 to 60 °C, preferably from 40 to 50 °C, and DRY1-2 may be done at a RH of from 20 to 50%, preferably from 30 to 40 %; or

H1-c) DRY1-1 may be done at a temperature of from 40 to 80°C, preferably from 50 to 65 °C,

DRY1-1 may be done at a RH of from 30 to 60 %, preferably from 40 to 50 %,
DRY1-2 may be done at a temperature of from 30 to 60 °C, preferably from 40 to 50 °C, and
DRY1-2 may be done at a RH of from 20 to 50%, preferably from 30 to 40 %;

or

H1-d) the time of DRY1-1 may be from 1 to 10 min, preferably from 2 to 8 min, in combination with anyone of embodiments H1-a), H1-b), or H1-c); or

H1-e) the time of DRY1-2 may be from 20 to 60 min, preferably from 30 to 55 min, in combination with anyone of embodiments H1-a), H1-b), or H1-c); or

H1-f) the time of DRY1-1 may be from 1 to 10 min, preferably from 2 to 8 min, and the time of DRY1-2 may be from 20 to 60 min, preferably from 30 to 55 min, in combination with anyone of embodiments H1-a), H1-b), or H1-c).

Anyone or more of the embodiments H11-a), H11-b), and H1 1-ab-c) may be combined with anyone or more of the embodiments H1-1), H1-2), H1-3) and H1-4).

Anyone or more of the embodiments H1-a), H1-b), H1-c), H1-d), H1-e) and H1-f) may be combined with anyone or more of the embodiments H1-1), H1-2), H1-3) and H1-4).

The viscosity of MELT2, in case that the first polymer is HPMC and the second polymer is HPMCAS, that is the HPMC-HPMCAS combination, may be from 100 to 2000 cP, preferably from 150 to 1000 cP, more preferably from 200 to 600 cP at the temperature of MELT2.

The temperature of MELT2, in case that the first polymer is HPMC and the second polymer is HPMCAS, that is the HPMC-HPMCAS combination, may be from 20 to 35 °C, preferably from 24 to 35 °C, more preferably 27 to 35 °C.

The mold pin, in case that the first polymer is HPMC and the second polymer is HPMCAS, that is the HPMC-HPMCAS combination, may have a temperature PINTEMPDIP2 for the DIP2 which is different from PINTEMPDIP1, the pin temperature for DIP1.

PINTEMPDIP2, in case that the first polymer is HPMC and the second polymer is HPMCAS, that is the HPMC-HPMCAS combination, may be from 20 to 50 °C, preferably from 25 to 50 °C.

In preferred embodiments the following parameters apply to a MELT2 being a HPMCAS melt when the first melt MELT1 is a HPMC melt, that is the HPMC-HPMCAS combination, i.e. in case that the first polymer is HPMC and the second polymer is HPMCAS:

H2-1) The viscosity of MELT2 may be from 100 to 2000 cP, preferably from 150 to 1000 cP, more preferably from 200 to 600 cP at the temperature of MELT2, and
the temperature of MELT2, in case that the first polymer is HPMC and the second polymer is HPMCAS, may be from 20 to 35 °C, preferably from 24 to 35 °C, more preferably 27 to 35 °C;
or
H2-2) the viscosity of MELT2 may be from 100 to 2000 cP, preferably from 150 to 1000 cP, more preferably from 200 to 600 cP at the temperature of MELT2, and PINTEMPDIP2 may be from 20 to 50 °C, preferably from 25 to 50 °C;
or
H2-3) the temperature of MELT2 may be from 20 to 35 °C, preferably from 24 to 35 °C, more preferably 27 to 35 °C, and PINTEMPDIP2 may be from 20 to 50 °C, preferably from 25 to 50 °C;
or
H2-4) the viscosity of MELT2 may be from 100 to 2000 cP, preferably from 150 to 1000 cP, more preferably from 200 to 600 cP at the temperature of MELT2,

the temperature of MELT2, in case that the first polymer is HPMC and the second polymer is HPMCAS, may be from 20 to 35 °C, preferably from 24 to 35 °C, more preferably 27 to 35 °C, and
PINTEMPDIP2 may be from 20 to 50 °C, preferably from 25 to 50 °C.

In any one of the embodiments regarding the process claims of the HPMC-HPMCAS combination above, the drying of the second polymer film on the mold pin, in case that the first polymer is HPMC and the second polymer is HPMCAS, may be done in one stage with one drying, DRY2.
DRY2 may be done at a temperature of from 20 to 70 °C, preferably from 25 to 60 °C.
DRY2 may be done at a RH of from 5 to 50%, preferably from 5 to 35 %.
The time of DRY2 may be from 20 to 50 min, preferably from 20 to 40 min.
In any one of the embodiments regarding the process claims of the HPMC-HPMCAS combination above, preferred embodiments for the drying DRY2 of the second polymer film on the mold pin, in case that the first polymer is HPMC and the second polymer is HPMCAS, are:

H2-a) DRY2 may be done at a temperature of from 20 to 70 °C, preferably from 25 to 60 °C, and
DRY2 may be done at a RH of from 5 to 50%, preferably from 5 to 35 %;
or
H2-b) the time of DRY2 may be from 20 to 50 min, preferably from 20 to 40 min, in combination with the embodiments H2-a);
or
H2-ab) a combination of H2-a) with H2-b).

Anyone or more of the embodiments H2-a), H2-b) and H2-ab) may be combined with anyone or more of the embodiments H2-1), H2-2), H2-3) and H2-4).
In case that the first polymer is HPMC and the second polymer is HPMCAS, that is the HPMC-HPMCAS combination, then

anyone or more of the embodiments of the dipping and drying of the first polymer HPMC, that is
anyone or more of embodiments H1-1), H1-2), H1-3), H1-4), H1-a), H1-b), H1-c), H1-d), H1-e), H1-f) and any combination thereof,
or
anyone or more of embodiments H1-1), H1-2), H1-3), H1-4), H11-a), H11-b), H11-ab) and any combination thereof, may be combined with anyone or more of the embodiments of the dipping and drying of the second polymer HPMCAS, that is with anyone or more of embodiments H2-1), H2-2), H2-3), H2-4), H2-a), H2-b) and H2-ab) and any combination thereof.

Embodiments of process claims for preparing a capsule with a combination of an inner layer of gelatin and an outer layer of HPMCAS by double dipping, first in a gelatin melt, that is MELT1 being a gelatin melt, and then in a HPMCAS melt, that is MELT2 being a HPMCAS melt, this combination is also called gelatin-HPMCAS combination in the following, are:

The viscosity of MELT 1, in case that the first polymer is gelatin and the second polymer is HPMCAS, that is gelatin-HPMCAS combination, may be from 300 to 1200 cP, preferably from 300 to 1100 cP, at the temperature of MELT 1. The temperature of MELT 1, in case that the first polymer is gelatin and the second polymer is HPMCAS, that is gelatin-HPMCAS combination, may be from 40 to 50 °C, preferably from 42 to 48 °C, even more preferably from 44 to 46 °C.

PINTEMPDIP1, in case that the first polymer is gelatin and the second polymer is HPMCAS, that is gelatin-HPMCAS combination, may be from 20 to 35 °C, preferably from 22 to 28 °C, even more preferably from 23 to 26 °C.

In preferred embodiments the following parameters apply to the gelatin-HPMCAS combination, i.e. in case that the first polymer is gelatin and the second polymer is HPMCAS:

G1-1) The viscosity of MELT1 may be from 300 to 1200 cP, preferably from 300 to 1100 cP, at the temperature of MELT1, and

the temperature of MELT 1 may be from 40 to 50 °C, preferably from 42 to 48 °C, even more preferably from 44 to 46 °C; or

G1-2) the viscosity of MELT1 may be from 300 to 1200 cP, preferably from 300 to 1100 cP at the temperature of MELT 1, and

PINTEMPDIP1 may be from 20 to 35 °C, preferably from 22 to 28 °C, even more preferably from 23 to 26 °C; or

G1-3) the temperature of MELT1 may be from 40 to 50 °C, preferably from 42 to 48 °C, even more preferably from 44 to 46 °C, and

PINTEMPDIP1 may be from 20 to 35 °C, preferably from 22 to 28 °C, even more preferably from 23 to 26 °C; or

G1-4) the viscosity of MELT1 may be from 300 to 1200 cP, preferably from 300 to 1100 cP at the temperature of MELT1,

the temperature of MELT1 may be from 40 to 50 °C, preferably from 42 to 48 °C, even more preferably from 44 to 46 °C, and

PINTEMPDIP1 may be from 20 to 35 °C, preferably from 22 to 28 °C, even more preferably from 23 to 26 °C.

In any one of the embodiments regarding the process claims of the gelatin-HPMCAS combination, the drying of the first polymer film on the mold pin, in case that the first polymer is gelatin and the second polymer is HPMCAS, may be done in one stage with one drying, DRYG1.

DRYG1 may be done at a temperature of from 20 to 30 °C, preferably of from 23 to 28 °C, more preferably of from 25 to 27 °C.

DRYG1 may be done at a RH of from 30 to 45 %, preferably of from 32 to 43 %, more preferably of from 34 to 42 %.

The time of DRYG1 may be from 20 to 90 min, preferably from 30 to 70 min, more preferably from 40 to 55 min.

In any one of the embodiments regarding the process claims of the gelatin-HPMCAS combination, preferred embodiments for the drying DRYG1 of the second polymer film on the mold pin, in case that the first polymer is gelatin and the second polymer is HPMCAS, are:

G1-a) DRYG1 may be done at a temperature of from 20 to 30 °C, preferably of from 23 to 28 °C, more preferably of from 25 to 27 °C, and

DRYG1 may be done at a RH of from 30 to 45 %, preferably of from 32 to 43 %, more preferably of from 34 to 42 %; or

G1-b) the time of DRYG1 may be from 20 to 90 min, preferably from 30 to 70 min, more preferably from 40 to 55 min, in combination with the embodiments G1-a); or

G1-ab) a combination of G1-a) with G1-b).

Anyone or more of the embodiments G1-a), G1-b) and G1-ab) may be combined with anyone or more of the embodiments G1-1), G1-2), G1-3) and G1-4).

The viscosity of MELT2, in case that the first polymer is gelatin and the second polymer is HPMCAS, that is gelatin-HPMCAS combination, may be from 200 to 700 cP, preferably from 200 to 500 cP at the temperature of MELT2.

The temperature of MELT2, in case that the first polymer is gelatin and the second polymer is HPMCAS, that is gelatin-HPMCAS combination, may be from 20 to 35 °C, preferably from 24 to 32 °C, more preferably 27 to 29 °C. In one embodiment, the temperature of MELT2 is 28 °C.

The mold pin, in case that the first polymer is gelatin and the second polymer is HPMCAS, that is gelatin-HPMCAS combination, may have a temperature PINTEMPDIPG2 for the DIP2 which is different from PINTEMPDIP1, the pin temperature for DIP1.

PINTEMPDIPG2, in case that the first polymer is gelatin and the second polymer is HPMCAS, that is gelatin-HPMCAS combination, may be from 25 to 30 °C, preferably from 27 to 29 °C.

In preferred embodiments the following parameters apply to the gelatin-HPMCAS combination, i.e. in case that the first polymer is gelatin and the second polymer is HPMCAS:

G2-1) The viscosity of MELT2 may be from 200 to 700 cP, preferably from 200 to 500 cP

at the temperature of MELT2, and
the temperature of MELT2 may be from 20 to 35 °C, preferably from 24 to 32 °C, more preferably 27 to 29 °C, most preferably 28 °C;

or
G2-2) the viscosity of MELT2 may be from 200 to 700 cP, preferably from 200 to 500 cP

at the temperature of MELT2, and
PINTEMPDIPG2 may be from 25 to 30 °C, preferably from 27 to 29 °C;

or
G2-3) the temperature of MELT2 may be from 20 to 35 °C, preferably from 24 to 32 °C, more preferably 27 to 29 °C, most preferably 28 °C, and
PINTEMPDIPG2 may be from 25 to 30 °C, preferably from 27 to 29 °C;
or
G2-4) the viscosity of MELT2 may be from 200 to 700 cP, preferably from 200 to 500 cP at the temperature of MELT2,

the temperature of MELT2 may be from 20 to 35 °C, preferably from 24 to 32 °C, more preferably 27 to 29 °C, most preferably 28 °C, and
PINTEMPDIPG2 may be from 25 to 30 °C, preferably from 27 to 29 °C.

In any one of the embodiments regarding the process claims of the gelatin-HPMCAS combination, the drying of the first polymer film on the mold pin, in case that the first polymer is gelatin and the second polymer is HPMCAS, may be done in one stage with one drying, DRYG2.

DRYG2 may be done at a temperature of from 25 to 30 °C, preferably of from 27 to 29 °C.

DRYG2 may be done at a RH of from 5 to 20 %, preferably of from 8 to 15 %.

The time of DRYG1 may be from 5 to 45 min, preferably from 10 to 30 min, more preferably from 15 to 25 min.

In any one of the embodiments regarding the process claims of the gelatin-HPMCAS combination, preferred embodiments for the drying DRYG1 of the second polymer film on the mold pin, in case that the first polymer is gelatin and the second polymer is HPMCAS, are:

G2-a) DRYG2 may be done at a temperature of from 25 to 30 °C, preferably of from 27 to 29 °C, and
DRYG2 may be done at a RH of from 5 to 20 %, preferably of from 8 to 15 %;
or
G2-b) the time of DRYG1 may be from 5 to 45 min, preferably from 10 to 30 min, more preferably from 15 to 25 min;
G2-ab) a combination of G2-a) with G2-b).

Anyone or more of the embodiments G2-a), G2-b) and G2-ab) may be combined with anyone or more of the embodiments G2-1), G2-2), G2-3) and G2-4).

In case that the first polymer is gelatin and the second polymer is HPMCAS, that is the gelatin-HPMCAS combination, then

anyone or more of the embodiments of the dipping and drying of the first polymer gelatin, that is anyone or more of embodiments G1-1), G1-2), G1-3), G1-4), G1-a), G1-b), G1-ab) and any combination thereof,
may be combined with anyone or more of the embodiments of the dipping and drying of the second polymer HPMCAS, that is with anyone or more of embodiments G2-1), G2-2), G2-3), G2-4), G2-a), G2-b) and G2-ab) and any combination thereof.

Any GELSYS and any FURTHERSUBST may be comprised in MELT1, in MELT2 or in both.

Any FURTHERSUBST and any GELSYS may be comprised in MELT1 in an amount based on the amount of WATSOLPOL, or in MELT2 in an amount based on the amount of ENTPOL respectively, which is equal to the desired amount in the inner or outer layer respectively.

Further subject of the invention is CAPSSHELL obtainable by DOUBLEDIP;
with CAPSSHELL and with DOUBLEDIP as defined herein, also with all their embodiments.
Further subject of the invention is CAPSSHELL filled with a formulation FILLFORM, wherein

FILLFORM comprises an active ingredient ACTINGR, ACTINGR is selected from the group of active pharmaceutical ingredient, pharmaceutical dosage form, medicament, live biotherapeutic product, and nutritional product;
with CAPSSHELL as defined herein, also with all its embodiments.

Live biotherapeutic product may for example be a microbiome.
One use of CAPSSHELL is the delivery of live biotherapeutic products to the intestine; the enteric properties of CPASSHELL allow for the passage of the live biotherapeutic products in CAPSSHELL through the stomach without release of the content of CAASSHELL; the release happens only in the intestine after CPASSHELL has travelled through the stomach.
FILLFORM may have the form of a powder, a caplet or a tablet.
Further subject of the invention is the use of CAPSSHELL for filling with FILLFORM; with CAPSSHELL and with FILLFORM as defined herein, also with all its embodiments. Further subject of the invention is the use of CAPSSHELL filled with FILLFORM for oral intake; with CAPSSHELL and with FILLFORM as defined herein, also with all its embodiments.
FILLFORM may comprise ACTINGR in an amount from 0.05 to 100 wt%, preferably from 0.5 to 90 wt%, more preferably from 1 to 50 wt%, even more preferably from 5 to 30 wt%, the wt% being based on the dry weight of FILLFORM.
In one embodiment the inner layer of CAPSHELL does not contain ACTINGR, which means that ACTINGR is not loaded throughout the inner layer of CAPSHELL.

## EXAMPLES

### Test methods, materials and further abbreviations used in this specification

All the examples except Example 5 were done in capsule size 2. STD target weights (standard target weights, in the tables abbreviated with STD-T-W) of size 2 capsules was predefined to be:

capsule body, also called body: STD-T-W 37.5 mg
capsule cap, also called cap: STD-T-W 23.5 mg

For size 0 in Example 5 the respective values are:
capsule body, also called body: STD-T-W 60 mg
capsule cap, also called cap: STD-T-W 36 mg

| | |
|---|---|
| APAP | acetaminophen, CAS 103-90-2, SigmaAldrich No. A5000, grade meets USP testing specifications, 98.0 to 102.0 %, powder |
| AS-HG | HPMCAS-HG |
| Film Applicator | For film casting any commercially available film casting equipment, such as COATMASTER 510 XL from Erichsen, or any in-house developed equipment can be used. Depending on the parameters of the melt, such as viscosity, type of polymer, concentration and the like, and depending on the equipment used, appropriate conditions need to be chosen, which is known to the skilled person in the art, in order to cast a film with a desired thickness. |
| CAP | Eastman™ C-A-P Cellulose Ester NF (Cellulose Acetate Phthalate, NF), Eastman Chemical International GmbH, 6303 Zug, Switzerland, Specifications: Acetyl 21.5-26 %; Free Acid as Phthalic Acid 3.0 wt % max; Moisture 5.0 wt % max; Phthalyl 35 %; Viscosity @ 25 °C 45-90 cP (Centipoise, 15 % C-A-P in an acetone solution (68 cP = 80.5 centistokes) |
| gelatin | a grade suitable for capsule manufacture |
| HPMC | Hypromellose Type 2906, 5 mPas, Tylopur 65SH-5, SE Tylose GmbH, 65203 Wiesbaden, Germany |
| HPMCAS-HG | Hydroxypropyl methylcellulose acetate succinate, hypromellose acetate succinate, HG, grade HG, |

with a succinoyl content of 7.6%. Aqoat AS-HG, Shin-Etsu Chemical Co., Ltd., Tokyo, Japan, Further details are given in Table 27

HPMCAS-LG     Hydroxypropyl methylcellulose acetate succinate, hypromellose acetate succinate, LG, grade LG, with a succinoyl content of 14.6% Aqoat AS-LG, Shin-Etsu Chemical Co., Ltd., Tokyo, Japan, Further details are given in Table 27

HPMCAS in form of AQOAT® HG (also called AS-HG) and LG (also called AS-LG) were purchased from Shin-Etsu Chemical Co., Ltd. (Tokyo, Japan). The letters L, M, and H specify the grade and distinguish the contents of acetyl and succinoyl groups. The Letter G represents granular grade with a Mean Particle Size of 1 mm, a letter F instead of a G would represent micronized grade with a Mean Particle Size of 5 micrometer. Various contents and parameters of these grades are given in Table 27 (source: Shin-Etsu Chemical Co., Ltd.).

| Table 27 | | | | | | |
|---|---|---|---|---|---|---|
| Grade | Viscosity (mPa*s) (a) | Methoxy content [wt%](c) | Hydroxypropoxy content [wt%](c) | Acetyl content [wt%] range/ preferred (c) | Succinoyl content [wt%] range/preferred (c) | Tg [°C] (b) |
| L | 2.4 to 3.6 | 20 to 24 | 5 to 9 | 5 to 9 / 6 | 14 to 18 / 15 | 122 |
| M | 2.4 to 3.6 | 21 to 25 | 5 to 9 | 7 to 11/8 | 10 to 14/11 | 122 |
| H | 2.4 to 3.6 | 22 to 26 | 6 to 10 | 10 to 14 / 12 | 4 to 8 / 6 | 122 |
| (a) Viscosity of 2 w/w % solution of sodium hydroxide aqueous solution at 20 °C<br>(b) Tg of the HPMCAS was determined by DSC experiment under the following test condition:<br>Equipment: DSC Q2000 (TA Instruments. Japan)<br>Heating rate: 10 °C/min<br>Referred to the second heating run<br>$N_2$ gas atmosphere<br>Sample size 3 mg<br>(c) the wt % based on the weight of the HPMCAS | | | | | | |

LOD     Loss on Drying; the LOD is a back-weighing application to determine the amount of volatile matter in films, tablets, capsules, or any bulky material. Samples are weighed before and after treatment, and the weight difference is measured.
The LOD may be determined by drying the capsules (bodies and caps) or the film at 105 °C overnight and determining the weight before and after drying:
W: capsule weight before drying overnight at 105 °C
Wd: capsule weight after drying overnight at 105 °C

$$LOD = (W - Wd) / W$$

RH     relative humidity

RT     room temperature

STD-T-W     STD target weight, standard target weight. STD target weight is dependent on and is predefined for a given capsule size The sum of the STD-T-W of the cap and of the STD-T-W of the body is the STD capsule weight

$TiO_2$     Titanium Dioxide, Kronos Canada, Pharma grade, which can used for capsules

W     in the tables "W" is used as an abbreviation for "weight"

%     all % values are wt%, if not stated otherwise

**Tube test:** The capsule's fracture and elasticity behavior can be measured by its resistance to a crash or impact test with a tube tester developed in-house by Capsugel, now Lonza Ltd, Visp, Switzerland. In essence capsules are first subjected to storing for 5 days at chosen relative humidity or humidities (e.g. 2.5, 10, 16, 23, 33, 45, or 66% RH). After that, a capsule is layed horizontally on a flat surface and a weight of e.g. 100 gram is dropped from the inside of a tube that is placed over the capsule to be tested. The tube may have an internal diameter of 25.5 mm and the weight may have a diameter of 24.5 mm. The tube may have a height of 80 mm, that is the distance the weight falls down. A sample size of 50 capsules may be chosen wherein each capsule is tested individually. The number of broken bodies, caps or whole capsules (body and cap) at the different relative humidities is counted to calculate the fracture rate and elasticity behavior.

**Capsule top compression test:** This test is to evaluate the capsule top strength by top punch force or top punch energy which is the force or energy required to compress (vertically) the capsule (body or cap) top by 0.55 mm. Capsule top strength also depends upon the water content of the capsule hence the relative humidity of the storage period (24 hours to 5 days) needs to be taken into account. Generally, tested capsules are stored for example at 23% RH, 33% RH and even at 45% RH. Preferably an Instron Universal Testing Machine Series 5960 (number 5965) can be used using a 1 kN cell (N° 2580-106) for testing the force needed for compression of the body, cap or whole capsule.

**(Pre)lock force measurement**: Testing can be performed using a device incorporating a commercially available digital force gauge (e.g. Shimpo - USA). The body of the capsule is fixed (preferably with a needle or with air suction) in the holder, and the force required to remove the cap, at constant rate preferably at 70 mm/s, under prelock (where applicable) or lock conditions is recorded.

**Dissolution for enteric dosage**: This test measures the dissolution of enteric capsules in appropriate (acidic) media:

- Media and Test in accordance with EUROPEAN PHARMACOPOEIA 7.0 - 2.9.3 or 7.5 - 2.9.3, Dissolution test for solid dosage forms - Delayed-release solid dosage forms.

- Media and Test in accordance with USP Method A (as further detailed herein, see for example above in "Abbreviations and definitions")

The tests may be performed manually on dissolution bath type VanKel VK 7000 or other. The tests may be performed with a Paddle Apparatus (apparatus 2), preferably wherein the paddles are run at 50 RPM and wherein the distance between said paddle and the bottom of the dissolution bath vessel is about 2.5 +/- 0.2 cm.

The tests may be performed at pH 1.2 or at pH 6.8 as for example described in Method A, or it may be performed at any other pH, preferably at any other pH between pH 1.2 and pH 6.8, for example pH 4.5 may be of interest; the desired pH environment and test solution being provided by a respective aqueous buffer.

Dosage measurement may be performed with commercially available UV spectrophotometer, such as type Lambda 25, Lambda 35 or Uvikon XL, BioTek Instruments.

- Also a suitable test procedure for dissolution properties of the capsules is as follows: USP Dissolution Apparatus 2 (paddle) is used, dissolution media: simulated gastric fluid at pH 1.2 0.1 N HC1 for 2 h then simulated intestinal fluid at pH 6.8 with $Na_3PO_4$; Test conditions: fluid kept at 37°C, paddle vessel (USP/NF) of cylindrical form with spherical end; rotation speed 50 rpm; dissolution liquid volume is 750 ml. Test capsules are filled with 380 mg of acetaminophen. Capsules are then placed into the vessel which is placed in the simulated gastric fluid for 2h. Subsequently, 250 ml of 0.20 M tribasic sodium phosphate are added to simulated intestinal fluid pH 6.8. UV ($\lambda$ = 300 nm) is used to quantify dissolved acetaminophen (as % of filled amount) in the dissolution media. Measures are made every 15 min when in the simulated gastric fluid and every 3 min in the simulated intestinal fluid.

**Dissolution in demineralized water**: Dissolution of the capsules in demineralized water can be measured similarly to the dissolution test for acidic media as indicated above under "Dissolution for enteric dosage", wherein demineralized water is used (at 37 °C for 2 h duration maximum) instead of a buffer with a certain pH.

**Film tensile test**: The determination of mechanical properties of film specimen by tensile test is performed using an Instron testing device (e.g. Instron Universal Testing Machine Series 5960 (number 5965), using a 1 kN cell (N° 2580-106), the thickness of the film can be determined by commercial gauge, for example offered by Mitutoyo.

For testing, sample of 20 film strips (width 12.7 mm, length about 80 mm) are placed between the clamps of the instrument and the thickness of the film is measured and the deformation at break (a measure for changes in form or shape of the film, it can also be called elongation at break) is measured.

**Impact energy for fracture on film**: This test is to evaluate impact resistance of films in gelatin or other material. Films are prepared by casting (with Film Applicator), possible film thicknesses may be 100 micrometer +/- 15 micrometer or else, and after drying, films are cut into strips (e.g. 50 mm x 30 mm or 40 mm x 25 mm) and stored (equilibrated) to various relative humidity conditions (e.g. 2.5, 10, 16, 23, 33, 45, or 66% RH e.g. during 5 days). Film thickness is measured for each piece of film e.g. using a micrometer gauge (e.g from Mitutoyo) and values are recorded before storage in desiccators. Film strips are clamped in a sample holder with a window of e.g. 7 mm x 16 mm. The energy needed to break the film is then defined using a Mouton-Charpy pendulum. In essence, the energy is gradually increased up to the point where the film breaks and the impact resistance is expressed as the ratio of energy par sample thickness (mJ/mm).

A similar test can be performed on capsules as such a measurement of their resistance to impact using an impact tester. Capsule samples are equilibrated to various relative humidity conditions by storing in desiccators for a minimum of 5 days (2.5 - 10 - 16 - 23 - 33 - 45 - 66 %RH). Micrometer gauges are used for measuring the thickness of the impact zone of capsule bodies (e.g. Mitutoyo mode IDC-112B) or caps (e.g. from Käffer). In essence, the energy needed to break the capsule body or cap is measured by gradually increasing the energy of impact until the point that the body or cap breaks by being hit by an impact tip, and the impact resistance is expressed as the ratio of energy par sample thickness (mJ/mm).

**Piercing time measurement test**: This test is measuring the time required to pierce a disc of tested film by dissolution fluid, e.g. using a bowl from a dissolution bath type VanKel VK 7000 or other device. Depending on the film composition and the used fluid, piercing time will correspond to a dissolution time or to a breaking time of the film.

In essence film discs are stamped out with a diameter of about 18 mm, when possible with a thickness that should be about 100 $\mu$m. The thickness of the discs L is measured ($\mu$m). The film disc is then positioned in the cap of a dissolution cell with a dissolution window of about 14 mm diameter. This dissolution cell is immersed in a flask containing the dissolution fluid at 37°C and the chronometer is started. With the dissolution progressing, the film becomes thinner and thinner and will at a certain time be "pierced" by the dissolution fluid. If the film is not soluble in the used dissolution fluid, it could also break under the pressure of the air dilatation occurring inside the bottle. The time required for dissolution fluid to fill half of the bottle is recorded as PIERCING TIME (Tp). A piercing time normalized to a thickness of 100 $\mu$m (Tp.c) is calculated by the formula:

$$Tp.c = Tp * 100^2 / L^2$$

with:

L being: Thickness ($\mu$m)
Tp being: Piercing time
Tp.c being: Piercing time normalized to a thickness of 100 $\mu$m

The average Tp.c is calculated with the 10 more representative results among the 15 recorded.

**Puncture test**: This test is used to evaluate puncture resistance of films. Films are prepared by casting (with Film Applicator) and tested on Texture Analyzer device. This test can be performed on capsule wall fragments as well if the material has been purposely cut and stretched out prior to the test. After casting and drying, films are cut into strips (30 mm x 30 mm), then equilibrated to various relative humidity conditions (e.g. 2.5, 10, 16, 23, 33, 45, or 66% RH e.g. during 5 days). The film thickness is measured per strip before test. Next, the Texture Analyzer apparatus is equipped with its ball shaped probe (e.g. 5 mm diameter, standard probe P5/S). A piece of film is clamped between 2 holed metal plates equipped with 4 fixation screws (preferred diameter of hole is 10 mm). The initial position of the probe is about 5 cm above the sample. During the test, the probe goes down, perforates the film strip (or capsule body part) and comes back to the initial position. The corresponding curve is traced and the area below the peak is calculated as a measure of the puncture resistance of the film or capsule shell. The force at break is determined to be the maximum force before breaking, the deformation at break is determined to be the distance that the ball shape probe travels from initially touching the film until breakage.

**Example 1: Delayed-release hard capsules by double dip based on HPMC and HPMCAS-LG**

**Description of First HPMC layer dip**

5 kg of HPMC solution were prepared by dispersing 1 kg HPMC in 4 kg water at 80 °C. After dispersing the dispersion was debubbled by reducing the stirring speed, then the dispersion was cooled to 15 °C for complete HPMC particle solubilization, achieving a HPMC solution without bubbles.

Then the HPMC solution was poured into a dipping dish of a hard capsule manufacturing pilot machine, and the HPMC solution in the dish was heated to 32 °C. This was the so called first melt.

The first layer dipping was performed according to a thermal gelling process by using preheated hot pins (70 °C) and hot drying air (65 °C with 40 % RH for ca. 40 min).

The viscosity of the HPMC solution in the dish was adjusted by addition of water to achieve the desired weight of the solution that is picked-up on the pins by the dip, thus controlling the weight of the first layer, that weight is called the pick-up weight. The pick-up weight is determined by weighing the dipping bar before and after a dip. The desired pick-up weight again is set in view of the intended and predefined weight of the first layer of the capsules, this intended and predefined weight was for example 70 wt% of standard target weight STD-T-W, that is

$$body = 37.5 \times 70 \% = 26.3 \ mg$$

$$cap = 23.5 \times 70 \% = 16.5 \ mg$$

The weights of the body and of the cap are determined by weighing them after drying, stripping from the pins and cutting to size.

For the purpose of viscosity adjustment the dipped bar was dried (hot drying air of 65 °C with 40 % RH for ca. 40 min), then stripped, the obtained bodies and caps were cut to the desired size, then the weight was determined to know the weight of the first layer of the bodies and caps, and the viscosity was adjusted accordingly in order to achieve the intended and predefined weight.

For preparing the dipped bars after having adjusted the viscosity, which were used for the second dip, the dipped bars were dried (hot drying air of 65 °C with 40 % RH for ca. 40 min) and were kept after drying in a non-stripped state. In parallel some of these bars were also stripped and the capsule bodies and caps were cut, then the bodies and caps were equilibrated at 22 °C and 50 % RH overnight and their weight was determined. This weight is the weight "W [mg]" of " 1st layer HPMC" reported in Table 1.

Also determined with these equilibrated capsules was the LOD. For this purpose the capsules (bodies and caps) were dried at 105 °C overnight and the weight was determined:

W: capsule weight before drying overnight at 105 °C
Wd: capsule weight after drying overnight at 105 °C

$$LOD = (W - Wd) \ / \ W$$

The LOD was 5.7 %

### Description of Second HPMCAS-LG layer dip

5 kg of HPMCAS-LG solution were prepared by dispersing 700 g HPMCAS-LG in 4300 g water at room temperature, then progressively adding 70 ml of 30 wt% aqueous ammonia solution (0.873 mol succinoyl and 1.11 mol $NH_3$ provides 1.27 molar equivalents of $NH_3$ based on molar amount of succinoyl residues of the HPMCAS-LG) under stirring to achieve total solubilization of HPMCAS-LG. The quantity of ammonia solution added was chosen to be just enough for completely dissolving the HPMCAS-LG particles.

14 g $TiO_2$ was added to the solution resulting in a suspension to provide for a white and opaque second layer. The obtained suspension containing the dissolved HPMCAS-LG and the suspended $TiO_2$ was debubbled overnight under vacuum of ca. 200 mbar at room temperature. The suspension containing the dissolved HPMCAS-LG and the suspended $TiO_2$ was poured into a dipping dish of a hard capsule manufacturing pilot machine, and the suspension in the dish was heated to 34 °C. This was the so called second melt.

The second dip was performed with a temperature of the bar of 30 °C with the dipped bars having the first layer and having been prepared as described under "First HPMC layer dip", after the second dip the double dipped bars were air dried at 30 °C at 7 % RH for ca. 20 min. Also for the second dip the viscosity of the suspension in the dish was adjusted by addition of water to achieve a final capsule weight equal or at least close to the STD target weight, this adjusting of the viscosity was done in the same way with these dried bars as described under "First HPMC layer dip".

For example when the intended weight of the first layer of the capsules had been predefined to be 70 wt% of standard target weight STD-T-W as stated under "First HPMC layer dip", the intended weight of the second layer of the capsules had been predefined to be 30 wt% of STD-T-W corresponding to the predefined 70 wt% of the first layer, that is

$$body = 37.5 \times 30 \% = 11.25 \ mg$$

$$cap = 23.5 \times 30\ \% = 7.05\ mg$$

In this way arriving at the STD-T-W of cap and of body was aimed at.

Those double dipped bars, which were used for the production of capsules used for testing delayed-release performance, were stripped after the drying, the bodies and caps were cut to size and were equilibrated at 50 % RH at 22 °C overnight and their weight was determined. This weight is the weight "W [mg]" of "Total" reported in Table 1. So "Total" is the weight of the 1st and the 2nd layer.

The weight "W [mg] of the "2nd layer HPMCAS-LG" as stated in Table 1 was calculated by:

W of 2nd layer = W of Total - W of 1st layer.

The W/STD W [%] in Table 1 is calculated by:

For the body:

1st layer W/STD-T-W [%] = W of 1st layer/STD-T-W = W of 1st layer/37.5 mg
2nd layer W/STD-T-W [%] = W of 2nd layer/STD-T-W = W of 2nd layer/37.5 mg
Total W/STD-T-W [%] = W of Total/STD-T-W = W of Total/37.5 mg.

For the cap:

1st layer W/STD-T-W [%] = W of 1st layer/STD-T-W = W of 1st layer/23.5 mg
2nd layer W/STD-T-W [%] = W of 2nd layer/STD-T-W = W of 2nd layer/23.5 mg
Total W/STD-T-W [%] = W of Total/STD-T-W = W of Total/23.5 mg.

For the examples the first and second layer dips were done while choosing different viscosities of the solution (first dip) and the suspension (second dip) in the dish to achieve different combinations of weights of the two layers, while always aiming at obtaining the same STD target weight or at least aiming at arriving close to the STD target weight.

Table 1 gives the details for the produced capsules.

| | Table 1 | | | | |
|---|---|---|---|---|---|
| Ex | Capsule Shell | | 1st layer HPMC | 2nd layer HPMCAS-LG (calculated) | Total |
| 1A | Cap | W [mg] | 18.0 | 7.4 | 25.4 |
| | | W/STD-T-W [%] | 76.6 | 31.5 | 108.1 |
| | Body | W [mg] | 26.8 | 12.0 | 38.8 |
| | | W/STD-T-W [%] | 71.5 | 32.0 | 103.5 |
| 1B | Cap | W [mg] | 18.8 | 5.2 | 24.0 |
| | | W/STD-T-W [%] | 80.0 | 22.1 | 102.1 |
| | Body | W [mg] | 29.9 | 9.2 | 39.1 |
| | | W/STD-T-W [%] | 79.7 | 24.5 | 104.3 |
| 1C | Cap | W [mg] | 20.4 | 5.1 | 25.5 |
| | | W/STD-T-W [%] | 86.8 | 21.7 | 108.5 |
| | Body | W [mg] | 33.7 | 6.50 | 40.1 |
| | | W/STD-T-W [%] | 89.9 | 17.3 | 106.9 |

The LOD of the second layer was determined with respective films:

Films were made by casting on a support films with the second melt which was used for the second dip. The films were dried at room temperature, After drying the films were removed from the support, equilibrated at 50 % RH at 22 °C overnight and their weight was determined. Also determined with these equilibrated films was the LOD. For this purpose the films were dried at 105 °C overnight and the weight was determined:

W: film weight before drying overnight at 105 °C

Wd: film weight after drying overnight at 105 °C

$$LOD = (W - Wd) / W$$

The LOD was 6.72 %

**Capsule evaluation of their delayed-release performance**

As neutralized HPMCAS aqueous solution is used for the second layer dip, the second layer is water soluble.

For an optimal delayed-release performance and avoiding any ammonia smell in a capsule container in big quantity, a post-treatment is performed to reduce the ammonia content to minimum.

Post-Treatment:

The capsules produced according to the description "Second HPMCAS-LG layer dip" were kept at 60 °C overnight, followed by a second equilibration at 50 % RH at 22 °C for 1 day before the evaluation.

The capsules were evaluated by in-vitro dissolution test Method A USP for delayed-release dosage form. For this purpose the capsules were filled with acetaminophen (APAP) and closed. The dissolution test was performed at 37 °C first in simulated gastric medium (pH 1.2) for 120 min, then in simulated intestinal fluid (pH 6.8) for 1 h and 15 min as described in the Method A USP. A sinker was used to keep the capsule on the bottom of the dissolution vessel, 50 rpm stirring were applied. 6 capsules were tested of each example 1A, 1B and 1C and the average of their values is reported.

The results show that all produced capsules have good delayed-release performance.

Even the capsules with the lowest HPMCAS-LG layer weight ratio "W/STD-T-W [%]" of ca. 19.5 %, these 19.5 % being the average of the lowest weight ratio of the cap of 21.7 % and of the body of 17.3 %, showed good delayed-release performance.

Details are given in Table 2. "max " in Table 2 denotes the value of the one sample with the highest value.

| | Table 2 | Example 1A | Example 1B | Example 1C |
| --- | --- | --- | --- | --- |
| pH | Time [min] | APAP dissolved [%] | APAP dissolved [%] | APAP dissolved [%] |
| 1.2 | 0 | 0.0 | 0.0 | 0.0 |
| 1.2 | 15 | 0.1 | 0.1 | 0.1 |
| 1.2 | 30 | 0.2 | 0.1 | 0.2 |
| 1.2 | 45 | 0.3 | 0.3 | 0.4 |
| 1.2 | 60 | 0.5 | 0.6 | 0.7 |
| 1.2 | 75 | 0.5 | 0.8 | 0.8 |
| 1.2 | 90 | 0.7 | 0.9 | 1.2 |
| 1.2 | 105 | 1.0 | 1.1 | 1.5 |
| 1.2 | 120 | **1.2 (max: 1.5)** | **1.3 (max: 1.8)** | **1.9 (max: 2.3)** |
| 6.8 | 135 | 42.3 | 46.4 | 42.1 |
| 6.8 | 150 | 73.2 | 78.7 | 68.6 |
| 6.8 | 165 | **86.5** | **89.3** | **83.5** |
| 6.8 | 180 | 93.1 | 93.7 | 93.1 |
| 6.8 | 195 | 96.1 | 96.0 | 96.5 |

All capsules showed the desired release of less than 10% of their content after 120 min at pH 1.2, and of more than 80% of their content after 45 min at pH 6.8.

Calculation of content:

| | | |
|---|---|---|
| First Layer: | 94.3 wt% HPMC | |
| | 5.7 wt% water (LOD) | |
| | (wt% based on weight of first layer) | |
| Second melt: | 700 g HPMCAS-LG | |
| | 14 g $TiO_2$ | |
| Second layer: | 91.45 wt% HPMCAS-LG = [93.28 wt % * 700 g / (700 + 14) g] | |
| | 1.83 wt% $TiO_2$ = [93.28 wt % * 14 g / (700 + 14) g] | |
| | 6.72 wt% water (LOD) | |
| | (wt% based on weight of second layer) | |

Table 9 and Table 10 give the wt% of the compositions, the values given in "%" in table 9 are wt% based on total weight, the values given in "%" in table 10 are wt% based on dry weight. "W" means weight.

| Ex | Table 9 | 1st layer | | | 2nd layer | | | |
|---|---|---|---|---|---|---|---|---|
| | | | | | LG | | | |
| | | W | LOD 5.7% | HPMC 94.3% | W (calc.) | LOD 6.72% | LG 91.45% | TiO2 1.83% |
| | | [mg] | [mg] | [mg] | [mg] | [mg] | [mg] | [mg] calc. |
| 1A | Cap | 18.0 | | | | | | |
| | Body | 26.8 | | | | | | |
| | Caspule | 44.8 | 2.6 | 42.2 | 19.4 | 1.3 | 17.7 | 0.4 |
| | | | | | | | | |
| 1B | Cap | 18.8 | | | 5.2 | | | |
| | Body | 29.9 | | | 9.2 | | | |
| | Caspule | 48.7 | 2.8 | 45.9 | 14.4 | 1.0 | 13.2 | 0.2 |
| | | | | | | | | |
| 1C | Cap | 20.4 | | | 5.1 | | | |
| | Body | 33.7 | | | 6.50 | | | |
| | Caspule | 54.1 | 3.1 | 51.0 | 11.6 | 0.8 | 10.6 | 0.2 |

| Ex | Table 10 | Total 1st+2nd | HPMC | | LG | | TiO2 |
|---|---|---|---|---|---|---|---|
| | | Dry W | W | | W | | W calc. |
| | | [mg] | [mg] | [%] | [mg] | [%] | [%] |
| 1A | Caspule | 64.2-2.6-1.3 = 60.3 | 42.2 | 70.0 | 17.7 | 29.4 | 0.6 |
| 1B | Caspule | 63.1-2.8-1.0 = 59.3 | 45.9 | 77.4 | 13.2 | 22.2 | 0.4 |
| 1C | Caspule | 65.7-3.1-0.8 = 61.8 | 51.0 | 82.5 | 10.6 | 17.2 | 0.3 |

**Example 2: Delayed-release hard capsules by double dip based on gelatin and HPMCAS-LG**

**First gelatin layer dip**

5 liters of 30 wt% gelatin aqueous solution at 50 °C, which was debubbled over night under vacuum of ca. 200 mbar at 50 °C, were poured into a dipping dish, then the solution in the dish was heated to 45 °C, the viscosity was adjusted according to the "Description of First HPMC layer dip" of example 1 in order to provide the desired weight of the first layer and the dip was performed according to the "Description of First HPMC layer dip" using pins with 26 °C and drying air (26 °C with 40 % RH for ca. 40 min).

The LOD of the capsules was determined as described under Example 1 to be 14.76 %.

**Second HPMCAS-LG layer dip**

The suspension containing the dissolved HPMCAS-LG and the suspended $TiO_2$ was prepared as described in example 1.

The viscosity of the suspension in the dish was adjusted as described in example 1. Once such viscosity was identified, the dipping of the bars with the first layer was done according to the "Description of Second HPMCAS-LG layer dip" of example 1, except that the temperature of the suspension was 28 °C, the temperature of the dipping bars was 28 °C and drying air of 28 °C at 12 % RH for 20 min was used.

The final weights of the bodies and caps were achieved and monitored as described in example 1.

For the examples the first and second layer dips were done while choosing different viscosities of the solution (first dip) and the suspension (second dip) in the dish to achieve different combinations of weights of the two layers, while always aiming at maintaining the same STD target weight or at least arriving close to the STD target weight.

Table 3 gives the details for the produced capsules.

| | Table 3 | | | | |
|---|---|---|---|---|---|
| Ex | Capsule | | 1st layer gelatin | 2nd layer HPMCAS-LG (calculated) | Total |
| 2A | Cap | W [mg] | 16.9 | 6.0 | 22.9 |
| | | W/STD-T-W [%] | 71.9 | 25.5 | 97.4 |
| | Body | W [mg] | 26.6 | 11.4 | 38 |
| | | W/STD-T-W [%] | 70.9 | 30.4 | 101.3 |
| 2B | Cap | W [mg] | 18.4 | 5.1 | 23.5 |
| | | W/STD-T-W [%] | 78.3 | 21.7 | 100.0 |
| | Body | W [mg] | 28.0 | 9.2 | 37.2 |
| | | W/STD-T-W [%] | 74.7 | 24.5 | 99.2 |
| 2C | Cap | W [mg] | 18.4 | 5.0 | 23.4 |
| | | W/STD-T-W [%] | 78.3 | 21.3 | 99.6 |
| | Body | W [mg] | 30.4 | 7.3 | 37.7 |
| | | W/STD-T-W [%] | 81.1 | 19.5 | 100.5 |
| 2D | Cap | W [mg] | 22.7 | 3.8 | 26.5 |
| | | W/STD-T-W [%] | 96.6 | 16.2 | 112.8 |
| | Body | W [mg] | 33.6 | 4.8 | 38.4 |
| | | W/STD-T-W [%] | 89.6 | 12.8 | 102.4 |

The LOD of films cast with the second melt was determined as described under Example 1 to be 6.72 %.

**Capsule evaluation of the delayed-release performance**

The produced capsules were post treated and evaluated for delayed-release performance as described in Example 1.

The results reveal a good delayed-release performance for the capsules.

Details are given in Table 4. "max " in Table 4 denotes the value of the one sample with the highest value.

| | Table 4 | Example 2A | Example 2B |
|---|---|---|---|
| pH | Time [min] | APAP dissolved [%] | APAP dissolved [%] |
| 1.2 | 0 | 0.0 | 0.0 |
| 1.2 | 15 | 0.0 | 0.2 |
| 1.2 | 30 | 0.1 | 0.2 |
| 1.2 | 45 | 0.2 | 0.4 |
| 1.2 | 60 | 0.4 | 0.6 |
| 1.2 | 75 | 0.7 | 0.8 |
| 1.2 | 90 | 1.0 | 1.1 |
| 1.2 | 105 | 1.3 | 1.5 |
| 1.2 | 120 | **1.6 (max: 1.8)** | **1.8 (max: 2.0)** |
| 6.8 | 135 | 63.1 | 57.7 |
| 6.8 | 150 | 84.5 | 86.6 |
| 6.8 | 165 | **92.0** | **96.8** |
| 6.8 | 180 | 96.1 | 99.4 |
| 6.8 | 195 | 98.5 | 99.9 |

All capsules showed the desired release of less than 10% of their content after 120 min at pH 1.2, and of more than 80% of their content after 45 min at pH 6.8.

Calculation of content:

| | |
|---|---|
| First Layer: | 85.24 wt% gelatin |
| | 14.76 wt% water (LOD) |
| | (wt% based on weight of first layer) |
| Second melt: | 700 g HPMCAS-LG |
| | 14 g $TiO_2$ |
| Second layer: | 91.45 wt% HPMCAS-LG = [93.28 wt % * 700 g / (700 + 14) g] |
| | 1.83 wt% $TiO_2$ = [93.28 wt % * 14 g / (700 + 14) g] |
| | 6.72 wt% water (LOD) |
| | (wt% based on weight of second layer) |

Table 11 and Table 12 give the wt% of the compositions, the values given in "%" in table 9 are wt% based on total weight, the values given in "%" in table 12 are wt% based on dry weight. "W" means weight.

| Ex | Table 11 | 1st layer | | | 2nd layer LG | | | |
|---|---|---|---|---|---|---|---|---|
| | | W | LOD 14.76% | gelatin 85.24% | W (calc.) | LOD 6.72% | LG 91.45% | TiO2 1.83% |
| | | [mg] | [mg] | [mg] | [mg] | [mg] | [mg] | [mg] calc. |
| 2A | Cap | 16.9 | | | 6.0 | | | |
| | Body | 26.6 | | | 11.4 | | | |
| | Caspule | 43.5 | 6.4 | 37.1 | 17.4 | 1.2 | 15.9 | 0.3 |
| | | | | | | | | |
| 2B | Cap | 18.4 | | | 5.1 | | | |

(continued)

| Ex | Table 11 | 1st layer | | | 2nd layer LG | | | |
|---|---|---|---|---|---|---|---|---|
| | | W | LOD 14.76% | gelatin 85.24% | W (calc.) | LOD 6.72% | LG 91.45% | TiO2 1.83% |
| | | [mg] | [mg] | [mg] | [mg] | [mg] | [mg] | [mg] calc. |
| | Body | 28.0 | | | 9.2 | | | |
| | Caspule | 46.4 | 6.8 | 39.6 | 14.3 | 1.0 | 13.1 | 0.2 |
| | | | | | | | | |
| 2C | Cap | 18.4 | | | 5.0 | | | |
| | Body | 30.4 | | | 7.3 | | | |
| | Caspule | 48.4 | 7.1 | 41.3 | 12.3 | 0.8 | 11.2 | 0.3 |
| | | | | | | | | |
| 2D | Cap | 22.7 | | | 3.8 | | | |
| | Body | 33.6 | | | 4.8 | | | |
| | Caspule | 56.3 | 8.3 | 48.0 | 8.6 | 0.6 | 7.9 | 0.1 |

| Ex | Table 12 | Total 1st+2nd | gelatin | | LG | | TiO2 |
|---|---|---|---|---|---|---|---|
| | | Dry W | W | | W | | W calc. |
| | | [mg] | [mg] | [%] | [mg] | [%] | [%] |
| 2A | Caspule | 60.9-6.4-1.2 = 53.3 | 37.1 | 69.6 | 15.9 | 29.8 | 0.6 |
| 2B | Caspule | 60.7-6.8-1.0 = 52.9 | 39.6 | 74.9 | 13.1 | 24.8 | 0.3 |
| 2C | Caspule | 60.7-7.1-0.8 = 52.8 | 41.3 | 78.2 | 11.2 | 21.2 | 0.6 |
| 2D | Caspule | 64.9-8.3-0.6 = 56.0 | 48.0 | 85.7 | 7.9 | 14.1 | 0.2 |

**Example 3: Delayed-release hard capsules by double dip based on gelatin and HPMCAS-HG**

**First gelatin layer dip**

The first dip was done as decried in Example 2

The LOD of the capsules was determined as described under Example 1 to be 14.76 %.

**Second HPMCAS-HG layer dip**

The suspension of HPMCAS-HG with $TiO_2$ was prepared as described in example 1 while using 700 g of HPMCAS-HG instead of HPMCAS-LG.

The second dip was done as described in example 2 while using this suspension of HPMCAS-HG with $TiO_2$ while using only 32 ml of 30 wt% aqueous ammonia solution instead of 70 ml (0.454 mol succinoyl and 0.507 mol $NH_3$ provides 1.12 molar equivalents of $NH_3$ based on molar amount of succinoyl residues of the HPMCAS-HG) under stirring to achieve total solubilization of HPMCAS-HG. The quantity of ammonia solution added was chosen to be just enough for completely dissolving the HPMCAS-HG particles.

Table 5 gives the details for the produced capsules.

| | Table 5 | | | 1st layer gelatin | 2nd layer HPMCAS-HG (calculated) | Total |
|---|---|---|---|---|---|---|
| Ex | Capsule | | | 1st layer gelatin | 2nd layer HPMCAS-HG (calculated) | Total |
| 3A | Cap | W [mg] | | 19.3 | 4.8 | 24.1 |
| | | W/STD-T-W [%] | | 82.1 | 20.4 | 102.6 |
| | Body | W [mg] | | 30.4 | 7.3 | 37.7 |
| | | W/STD-T-W [%] | | 81.1 | 19.5 | 100.5 |
| 3B | Cap | W [mg] | | 20.9 | 3.4 | 24.3 |
| | | W/STD-T-W [%] | | 88.9 | 14.5 | 103.4 |
| | Body | W [mg] | | 33.7 | 6.3 | 40.0 |
| | | W/STD-T-W [%] | | 89.9 | 16.8 | 106.7 |
| 3C | Cap | W [mg] | | 21.9 | 2.9 | 24.8 |
| | | W/STD-T-W [%] | | 93.2 | 12.3 | 105.5 |
| | Body | W [mg] | | 33.9 | 4.2 | 38.1 |
| | | W/STD-T-W [%] | | 90.4 | 11.2 | 101.6 |

The LOD of films cast with the second melt was determined as described under Example 1 to be 3.74 %.

In Example 3B, the viscosity of the gelatin melt of the first dip was 1000 cP at the temperature of the melt, and the viscosity of the HPMCAS-HG melt of the second dip was 450 cP at the temperature of the melt.

**Capsule evaluation of the delayed-release performance**

The produced capsules were off-gassed and evaluated for delayed-release performance as described in Example 1.

The results reveal that all produced capsules have a good delayed-release performance.

Even the capsules with the lowest HPMCAS-HG layer weight ratio "W/STD-T-W [%]" of 11.75 %, these 11.75 % being the average of the lowest weight ratio of the cap of 12.3 % and of the body of 11.2 %, showed good delayed-release performance.

Details are given in Table 6. "max " in Table 6 denotes the value of the one sample with the highest value.

| | Table 6 | Example 3A | Example 3B | Example 3C |
|---|---|---|---|---|
| pH | Time [min] | APAP dissolved [%] | APAP dissolved [%] | APAP dissolved [%] |
| 1.2 | 0 | 0.0 | 0.0 | 0.0 |
| 1.2 | 15 | 0.1 | 0.0 | 0.0 |
| 1.2 | 30 | 0.1 | 0.0 | 0.0 |
| 1.2 | 45 | 0.1 | 0.1 | 0.1 |
| 1.2 | 60 | 0.2 | 0.1 | 0.2 |
| 1.2 | 75 | 0.3 | 0.3 | 0.5 |
| 1.2 | 90 | 0.4 | 0.4 | 0.7 |
| 1.2 | 105 | 0.5 | 0.6 | 1.0 |
| 1.2 | 120 | **0.7 (max: 0.8)** | **0.8 (max 1.0)** | **1.3 (max: 1.4)** |
| 6.8 | 135 | 53.3 | 66.2 | 58.0 |
| 6.8 | 150 | 83.3 | 88.8 | 86.4 |
| 6.8 | 165 | **93.6** | **95.2** | **95.9** |

(continued)

| Table 6 | | Example 3A | Example 3B | Example 3C |
|---|---|---|---|---|
| pH | Time [min] | APAP dissolved [%] | APAP dissolved [%] | APAP dissolved [%] |
| 6.8 | 180 | 97.2 | 97.7 | 98.4 |
| 6.8 | 195 | 98.7 | 98.8 | 99.2 |

All capsules showed the desired release of less than 10% of their content after 120 min at pH 1.2, and of more than 80% of their content after 45 min at pH 6.8.

Calculation of content:

First Layer: 85.24 wt% gelatin
14.76 wt% water (LOD)
(wt% based on weight of first layer)

Second melt: 700 g HPMCAS-HG
14 g $TiO_2$

Second layer: 94.37 wt% HPMCAS-HG = [96.26 wt % * 700 g / (700 + 14) g]
1.89 wt% $TiO_2$ = [96.26 wt % * 14 g / (700 + 14) g]
3.74 wt% water (LOD)
(wt% based on weight of second layer)

Table 13 and Table 14 give the wt% of the compositions, the values given in "%" in table 9 are wt% based on total weight, the values given in "%" in table 14 are wt% based on dry weight. "W" means weight.

| Ex | Table 13 | 1st layer | | | 2nd layer LG | | | |
|---|---|---|---|---|---|---|---|---|
| | | W | LOD 14.76% | gelatin 85.24% | W (calc.) | LOD 3.74% | HG 94.37% | TiO2 1.89% |
| | | [mg] | [mg] | [mg] | [mg] | [mg] | [mg] | [mg] calc. |
| 3A | Cap | 19.3 | | | 4.8 | | | |
| | Body | 30.4 | | | 7.3 | | | |
| | Caspule | 49.7 | 7.3 | 42.4 | 12.1 | 0.5 | 11.4 | 0.2 |
| | | | | | | | | |
| 3B | Cap | 20.9 | | | 3.4 | | | |
| | Body | 33.7 | | | 6.3 | | | |
| | Caspule | 54.6 | 8.1 | 46.5 | 9.7 | 0.4 | 9.2 | 0.1 |
| | | | | | | | | |
| 3C | Cap | 21.9 | | | 2.9 | | | |
| | Body | 33.9 | | | 4.2 | | | |
| | Caspule | 55.8 | 8.2 | 47.6 | 7.1 | 0.3 | 6.7 | 0.1 |

| Ex | Table 14 | Total 1st+2nd | gelatin | | HG | | TiO2 |
|---|---|---|---|---|---|---|---|
| | | Dry W | W | | W | | W calc. |
| | | [mg] | [mg] | [%] | [mg] | [%] | [%] |
| 3A | Caspule | 61.8-7.3-0.5 = 54.0 | 42.4 | 78.5 | 11.4 | 21.1 | 0.4 |
| 3B | Caspule | 64.3-8.1-0.4 = 55.8 | 46.5 | 83.3 | 9.2 | 16.5 | 0.2 |
| 3C | Caspule | 62.9-8.2-0.3 = 54.4 | 47.6 | 87.5 | 6.7 | 12.3 | 0.2 |

**Example 4: Delayed-release hard capsules by double dip based on gelatin and CAP**

**First gelatin layer dip**

The first dip was done as decried in Example 2

The LOD of the capsules was determined as described under Example 1 to be 14.76 %.

**Second CAP layer dip**

The suspension of CAP with $TiO_2$ was prepared as described in example 1 while using 750 g of CAP instead of 700 g HPMCAS-LG, 15 g $TiO_2$ instead of 14 g $TiO_2$ and 130 ml of 30 wt% aqueous ammonia solution instead of 70 ml (1.590 mol succinoyl and 2.061 mol $NH_3$ provides 1.30 molar equivalents of $NH_3$ based on molar amount of phthalyl residues of the CAP) under stirring to achieve total solubilization of CAP.

The quantity of ammonia solution added was chosen to be just enough for completely dissolving the CAP particles.

The second dip was done as described in example 2 while using this suspension of CAP with $TiO_2$.

Table 7 gives the details for the produced capsules.

| | Table 7 | | | | |
|---|---|---|---|---|---|
| Ex | Capsule | | 1st layer | 2nd layer | Total |
| | | | gelatin | CAP (calculated) | |
| 4A | Cap | W [mg] | 18.7 | 4.8 | 23.5 |
| | | W/STD-T-W [%] | 79.6 | 20.4 | 100.0 |
| | Body | W [mg] | 32.0 | 7.9 | 39.9 |
| | | W/STD-T-W [%] | 85.3 | 21.1 | 106.4 |
| 4B | Cap | W [mg] | 20.1 | 3.8 | 23.9 |
| | | W/STD-T-W [%] | 85.5 | 16.2 | 101.7 |
| | Body | W [mg] | 32.1 | 5.7 | 37.8 |
| | | W/STD-T-W [%] | 85.6 | 15.2 | 100.8 |
| 4C | Cap | W [mg] | 21.4 | 2.7 | 24.1 |
| | | W/STD-T-W [%] | 91.1 | 11.5 | 102.6 |
| | Body | W [mg] | 33.5 | 4.0 | 37.5 |
| | | W/STD-T-W [%] | 89.3 | 10.7 | 100.0 |

The LOD of films cast with the second melt was determined as described under Example 1 to be 13.17 %.

**Capsule evaluation of the delayed-release performance**

The produced capsules were off-gassed and evaluated for delayed-release performance as described in Example 1.

The results reveal that all produced capsules have good delayed-release performance.

Even the capsules with the lowest CAP layer weight ratio "W/STD-T-W [%]" of 11 %, these 11 % being the average of the lowest weight ratio of the cap of 11.5 % and of the body of 10.7 %, showed good delayed-release performance.

Details are given in Table 8. "max " in Table 8 denotes the value of the one sample with the highest value.

|  | Table 8 | Example 4A | Example 4B | Example 4C |
|---|---|---|---|---|
| pH | Time | APAP dissolved | APAP dissolved | APAP dissolved |
|  | [min] | [%] | [%] | [%] |
| 1.2 | 0 | 0.0 | 0.0 | 0.0 |
| 1.2 | 15 | 0.0 | 0.0 | 0.0 |
| 1.2 | 30 | 0.2 | 0.0 | 0.1 |
| 1.2 | 45 | 0.2 | 0.1 | 0.2 |
| 1.2 | 60 | 0.2 | 0.2 | 0.3 |
| 1.2 | 75 | 0.4 | 0.3 | 0.4 |
| 1.2 | 90 | 0.4 | 0.4 | 0.6 |
| 1.2 | 105 | 0.4 | 0.5 | 0.6 |
| 1.2 | 120 | **0.6 (max:0.8)** | **0.5 (max: 0.6)** | **0.7 (max: 0.8)** |
| 6.8 | 135 | 62.6 | 47.9 | 52.4 |
| 6.8 | 150 | 90.2 | 88.0 | 85.6 |
| 6.8 | 165 | **96.8** | **98.3** | **96.0** |
| 6.8 | 180 | 98.9 | 99.9 | 99.0 |
| 6.8 | 195 | 99.5 | 100.0 | 99.6 |

All capsules showed the desired release of less than 10% of their content after 120 min at pH 1.2, and of more than 80% of their content after 45 min at pH 6.8.

Calculation of content:

| | |
|---|---|
| First Layer: | 85.24 wt% gelatin |
| | 14.76 wt% water (LOD) |
| | (wt% based on weight of first layer) |
| Second melt: | 750 g CAP |
| | 15 g $TiO_2$ |
| Second layer: | 85.13 wt% CAP = [86.83 wt % * 750 g / (750 + 15) g] |
| | 1.70 wt% $TiO_2$ = [86.83 wt % * 15 g / (750 + 15) g] |
| | 13.17 wt% water (LOD) |
| | (wt% based on weight of second layer) |

Table 15 and Table 16 give the wt% of the compositions, the values given in "%" in table 9 are wt% based on total weight, the values given in "%" in table 16 are wt% based on dry weight. "W" means weight.

| Ex | Table 15 | 1st layer | | | 2nd layer LG | | | |
|---|---|---|---|---|---|---|---|---|
| | | W | LOD 14.76% | gelatin 85.24% | W (calc.) | LOD 13.17% | CAP 85.13% | TiO2 1.70% |
| | | [mg] | [mg] | [mg] | [mg] | [mg] | [mg] | [mg] calc. |
| 4A | Cap | 18.7 | | | 4.8 | | | |
| | Body | 32.0 | | | 7.9 | | | |
| | Caspule | 50.7 | 7.5 | 43.2 | 12.7 | 1.7 | 10.8 | 0.2 |
| | | | | | | | | |
| 4B | Cap | 20.1 | | | 3.8 | | | |
| | Body | 32.1 | | | 5.7 | | | |
| | Caspule | 52.2 | 7.7 | 44.5 | 9.5 | 1.3 | 8.1 | 0.1 |
| | | | | | | | | |
| 4C | Cap | 21.4 | | | 2.7 | | | |
| | Body | 33.5 | | | 4.0 | | | |
| | Caspule | 54.9 | 8.1 | 46.8 | 6.7 | 0.9 | 5.7 | 0.1 |

| Ex | Table 16 | Total 1st+2nd | gelatin | | CAP | | TiO2 |
|---|---|---|---|---|---|---|---|
| | | Dry W | W | | W | | W calc. |
| | | [mg] | [mg] | [%] | [mg] | [%] | [%] |
| 4A | Caspule | 63.4-7.5-1.7 = 54.2 | 43.2 | 79.7 | 10.8 | 19.9 | 0.4 |
| 4B | Caspule | 61.7-7.7-1.3 = 52.7 | 44.5 | 84.4 | 8.1 | 15.4 | 0.2 |
| 4C | Caspule | 61.6-8.1-0.9 = 52.6 | 46.8 | 89.0 | 5.7 | 10.8 | 0.2 |

**Example 5: Double dipped hard capsules size 0: HPMC 1st layer and HPMCAS-HG 2nd layer**

HPMC melt for the first layer dipping is prepared as described in Example 1. The HPMC melt is filled into a dipping dish of a hard capsule manufacturing pilot machine with a temperature of 32 °C. The first layer making is based on the thermal gelling process, like example 1. The melt viscosity is adjusted to achieve a 1st layer weight equal to 85% of STD target weight of size 0 with size 0 dipping bars with the respective mold pins, that is 51 mg/body and 30.6 mg/cap. Other process conditions are like example 1.

HPMCAS-HG melt for the 2nd layer dipping is prepared as described in Example 3. The HMPCAS-HG melt is filled into a dipping dish of a hard capsule manufacturing pilot machine with a temperature of 28 °C. The melt viscosity is adjusted to achieve a 2nd layer weight equal to 15% of STD target weight of size 0 with size 0 dipping bars with the respective mold pins, that is 9 mg/body and 5.4 mg/cap.

After the identification of the melt viscosities of the HPMC melt and of the HPMCAS-HG melt to achieve the intended layer weight respectively, dippings are performed for the 1st layer. After withdrawing the mold pins from the HPMC melt the HPMC film of the 1st layer formed on the mold pins and was allowed to dry for 45 min at 50°C and a relative humidity of 40%, then these mold pins are used for a 2nd dipping for the 2nd layer without further waiting time. After withdrawing the pins from the HPMCAS-HG melt the HPMCAS-HG film formed on 1st layer on the pins and was allowed to dry for about 30 min drying at 40°C and a relative humidity of 20 %. Then the bodies and caps are stripped and cut into the desired length according to the specification of size 0. Thus the double dipped capsules are produced.

The viscosity of the HPMC melt of the first dip was 960 cP at the temperature of the melt, and the viscosity of the HPMCAS-HG melt of the second dip was 310 cP at the temperature of the melt.

The weights of each layer of the produced capsules are determined as described in examples 1, and reported in table 17.

| | Table 17 | | | | |
|---|---|---|---|---|---|
| Ex | Capsule Shell | | 1st layer | 2nd layer | Total |
| | | | HPMC | HPMCAS-HG (calculated) | |
| 5 | Cap | W [mg] | 30.4 | 5.7 | 36.1 |
| | | W/STD-T-W [%] | 84.4 | 15.6 | 100.3 |
| | Body | W [mg] | 53.0 | 8.8 | 61.8 |
| | | W/STD-T-W [%] | 88.3 | 14.7 | 103.0 |

The produced capsules were controlled one by one visually for possible shell crack defects formed during the drying steps. No cracks were found for all the bodies (N=500) and caps (N=350) produced.

Capsules are evaluated by dissolution test (Method A, USP). The results are shown in Table 18:

| | | Table 18 |
|---|---|---|
| | | Example 5 |
| pH | Time [min] | APAP dissolved [%] |
| 1.2 | 0 | 0 |
| 1.2 | 15 | 0.02 |
| 1.2 | 30 | 0.06 |
| 1.2 | 45 | 0.09 |
| 1.2 | 60 | 0.17 |
| 1.2 | 75 | 0.25 |
| 1.2 | 90 | 0.34 |
| 1.2 | 105 | 0.44 |
| 1.2 | 120 | **0.54 (max: 0.61)** |
| 6.8 | 135 | 12.01 |
| 6.8 | 150 | 49.41 |
| 6.8 | 165 | 80.32 |
| 6.8 | 180 | 94.65 |
| 6.8 | 195 | 99.04 |
| 6.8 | 210 | 99.53 |
| 6.8 | 225 | 99.81 |
| 6.8 | 240 | 99.88 |

All capsules showed the desired release of less than 10% of their content after 120 min at pH 1.2, and of more than 80% of their content after 45 min at pH 6.8.

The mechanical performance of the capsules were evaluated by a tube-test: A stainless steel cylinder weighing 100 g was allowed to fall from a height of 8 cm onto pre-j oined empty capsules one by one. The percentage of broken capsules is reported in Table 19. Before the evaluation, capsules are equilibrated at 22 °C/45 % RH & 22 °C/33 %RH, n = 50 respectively for 1 week.

| Table 19 | |
|---|---|
| **Equilibrium conditions** | **broken capsules [%]** |
| **45 %RH/22 °C** | 0 |
| **33 %RH/22 °C** | 0 |

All capsules have passed the tube test and show the required mechanical strength.

Calculation of content:

| | |
|---|---|
| First Layer: | 94.3 wt% HPMC |
| | 5.7 wt% water (LOD) |
| | (wt% based on weight of first layer) |
| Second melt: | 700 g HPMCAS-HG |
| | 14 g $TiO_2$ |
| Second layer: | 94.37 wt% HPMCAS-HG = [96.26 wt % * 700 g / (700 + 14) g] |
| | 1.89 wt% $TiO_2$ = [96.26 wt % * 14 g / (700 + 14) g] |
| | 3.74 wt% water (LOD) |
| | (wt% based on weight of second layer) |

Table 20 and Table 21 give the wt% of the compositions, the values given in "%" in table 20 are wt% based on total weight, the values given in "%" in table 21 are wt% based on dry weight. "W" means weight.

| Ex | Table 20 | 1st layer | | | 2nd layer HG | | | |
|---|---|---|---|---|---|---|---|---|
| | | W | LOD 5.7% | HPMC 94.3% | W (calc.) | LOD 3.74% | HG 94.37% | TiO2 1.89% |
| | | [mg] | [mg] | [mg] | [mg] | [mg] | [mg] | [mg] calc. |
| 5 | Cap | 30.4 | | | 5.7 | | | |
| | Body | 53.0 | | | 8.8 | | | |
| | Caspule | 83.4 | 4.8 | 78.6 | 14.5 | 0.5 | 13.7 | 0.3 |

| Ex | Table 21 | Total 1st+2nd | HPMC | | HG | | TiO2 |
|---|---|---|---|---|---|---|---|
| | | Dry W | W | | W | | W calc. |
| | | [mg] | [mg] | [%] | [mg] | [%] | [%] |
| 5 | Caspule | 97.9-4.8-0.5 = 92.6 | 78.6 | 84.9 | 13.7 | 14.8 | 0.3 |

**Example 6: Comparison of films based on HPMC**

The melts for preparing films were:
(a) HPMC: The melt was the HPMC melt for the first layer dipping as described and prepared in Example 5.
(b 1) and (b2) Double Layer
1st layer HPMC: The melt was the HPMC melt for the first layer dipping as described and prepared in Example 5.
2nd layer AS-HG: The melt was prepared by dispersing 20 kg of HPMCAS-HG powder into 180 kg of water at room temperature. In total, 800 g of ammonia 30% solution was added to dissolve all HPMCAS-HG particles. 408 g of TiO2, dispersed in an additional 3 kg of water, was added to opacify the melt.
Vacuum of up to 200 mbar was applied during one night to remove entrapped air in the melt. Composition:

HPMCAS-HG    9.8 wt%

(continued)

| | |
|---|---|
| Ammonia | 0.12 wt% |
| TiO2 | 0.20 wt% |
| Water | 89.88 wt% |

(c) Mixture of 85% HPMC with 15% AS-HG:

5000 g of mixture of 85% HPMC with 15% AS-HG was prepared by mixing 3725 g of HPMC melt, the one used for first layer dipping as described and prepared in Example 5, and 1275 g of HPMCAS-HG melt, the one used for the 2nd layer of the double layer films (b1) and (b2) in Example 6, under gentle anchor stirring at 28 °C, in proportions so that final weight content of dry HPMC is 85 wt% and final weight content of dry HPMCAS-HG is 15 wt%. The HPMC and HPMCAS-HG grades were respectively the same than the ones used for the HPMC and the HPMCAS-HG melts, as prepared in Example 5.

Calculation of content:

A polymer solids content of 19 wt% is considered for the HPMC melt and a polymer solids content of 9.8 wt% is considered for the HPMCAS-HG melt. Calculation of the exact quantity of HPMC melt and HPMCAS-HG melt to be mixed:

$$(19 \text{ wt\%} * W1) / (9.8 \text{ wt\%} * W2) = 85 \text{ wt\%}/15 \text{ wt\%}$$

and

$$W1 + W2 = 5000 \text{ g}$$

W1 and W2 meaning respectively the weight of HPMC melt to be mixed and the weight of HPMCAS-HG melt to be mixed.

Solving the above system of equations yields: W1 = 3725 g and W2 = 1275 g.

Several films were prepared by casting with Film Applicator, thicknesses of films are as given in Table 22.

| Table 22 | |
|---|---|
| **Film** | **Average film thickness** |
| | **[micrometer]** |
| (a) HPMC | 115 |
| (b1) Double Layer 1st layer: HPMC ca. 85 wt% 2nd layer: AS-HG ca. 15 wt% | 96 15 total 111 |
| (b2) Double Layer 1st layer: HPMC ca. 85% 2nd layer: AS-HG ca. 15% | 85 30 total 115 |
| (c) Mixture of 85% HPMC with 15% AS-HG | 104 |

(d) AS-HG films were not possible to be cast with Film Applicator: Films were impossible to be detach and the films were inhomogeneous, the maximum layer thickness that was obtainable with Film Applicator was ca. 30 micrometer. Instead, AS-HG films were prepared by pouring an AS-HG melt into a Petri dish.

The AS-HG melt was the HPMCAS-HG melt for the 2nd layer of the double layer films (b 1) and (b2) as prepared in Example 5.

The average film thickness was between 90 to 100 micrometer.

With the puncture test the force at break was determined; for this purpose the films were equilibrated at 22 °C 50% RH over night. Table 23 gives the results.

| Table 23 | | Puncture Test |
|---|---|---|
| Film | Average film | Force at break |
| | thickness [micrometer] | [N] |
| (a) HPMC | 115 | 60 |
| (b1) Double Layer | 111 | 60 |
| (c) Mixture | 104 | 36 |
| (d) AS-HG | 90 - 100 | 32 |

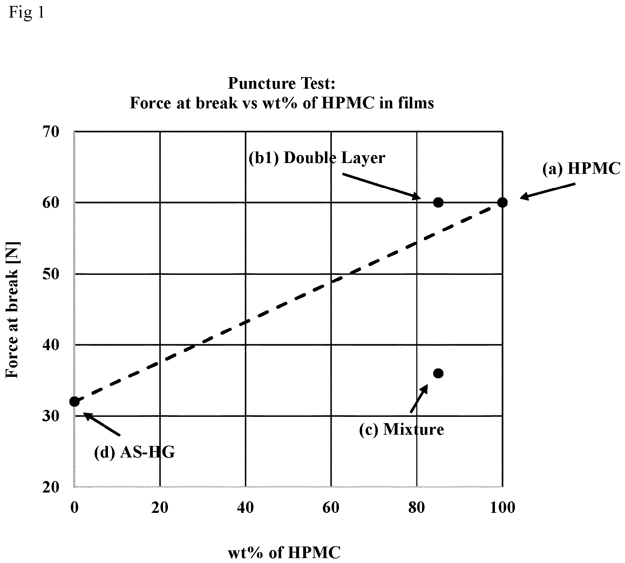

Figure 1 shows a graphical representation of the data of Table 23. The dashed line in figure 1 connecting the two extremes, AS-HG only and HPMC only, shows the expected values for the force at break in case of linear behavior. The Double Layer shows unexpected non-linear behavior.

With the tensile test the deformation at break was determined, table 24 gives the results.

| Table 24 | | Tensile Test |
|---|---|---|
| Film | Average film thickness [micrometer] | Deformation at break [%] |
| (a) HPMC | 115 | 14 |
| (b2) Double Layer | 115 | 17 |
| (c) Mixture | 104 | 10 |
| (d) AS-HG | 90 - 100 | 5 |

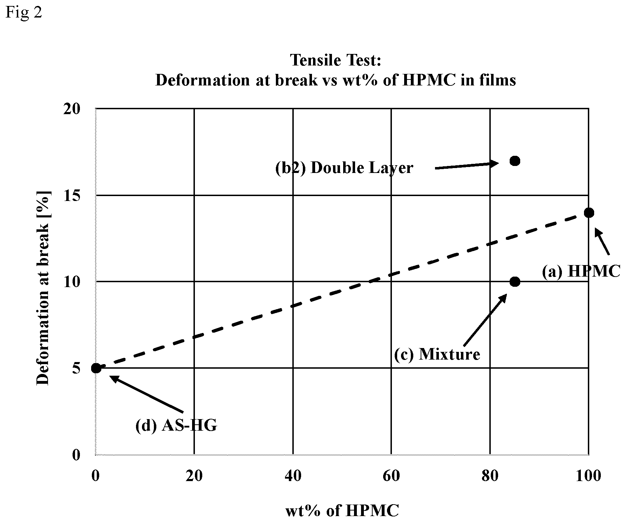

Figure 2 shows a graphical representation of the data of Table 24. The dashed line in Fig 2 connecting the two extremes, AS-HG only and HPMC only, shows the expected values for the deformation at break in case of linear behavior. The Double Layer shows unexpected non-linear behavior.

The test results of the puncture test and of the tensile test show unexpected non-linear improvements of the double layered film compared to films with similar thickness but with only one polymer and to films with similar thickness and two polymers but wherein the two polymers are not separated in individual layers but are present as a mixture in one layer.

**Example 7: Comparison of films based on gelatin**

The melts for preparing films were:

(e) Gelatin: The melt was the gelatin melt for the first layer dipping as described and prepared in Example 3B.

(f) Double Layer

1st layer: gelatin: The melt was the gelatin melt for the first layer dipping as described and prepared in Example 3B. 2nd layer: AS-HG: The melt was the HPMCAS-HG melt for the 2nd layer of the double layer films (b1) and (b2) as prepared in example 6.

Several films were prepared by casting with Film Applicator, Table 25 gives the thickness of the films.

| Table 25 | |
|---|---|
| Film | Average film thickness [micrometer] |
| (e) Gelatin | 100 |
| (f) Double Layer 1st layer: gelatin ca. 85 wt% 2nd layer: AS-HG ca. 15 wt% | 90 10 total 100 |

It was not possible to prepare a film containing a mixture of 85 wt% gelatin and 15 wt% AS-HG, the melt shows phase separation, aggregation and even solid chunks depending of the of temperature of 28 °C or 55 °C.

(g) AS-HG films were not possible to be cast with Film Applicator: Films were impossible to detach and increasing of the door opening led to inhomogeneous films, the maximum layer thickness that was obtainable with Film Applicator was ca. 30 micrometer. Instead, AS-HG films were prepared by pouring a AS-HG melt into a Petri dish. The AS-HG melt was the HPMCAS-HG melt prepared for the 2nd layer of the double layer films (b 1) and (b2) as described in Example 6.

The average film thickness was between 90 to 100 micrometer.

With the tensile test the deformation at break was determined, table 26 gives the results.

| Table 26 | | Tensile Test | |
|---|---|---|---|
| Film | Average film thickness [micrometer] | Elastic Modulus [MPa] | Stress at break [MPa] |
| (e) gelatin | 100 | 2228 | 78 |
| (f) Double Layer | 100 | 2402 | 87 |
| (g) AS-HG | 90 - 100 | 1549 | 47 |

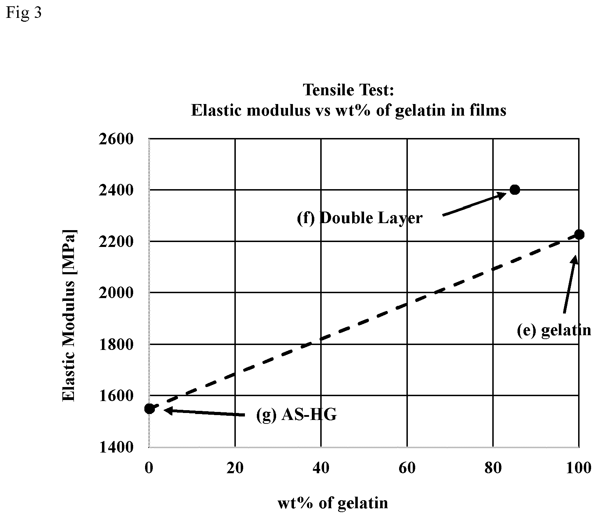

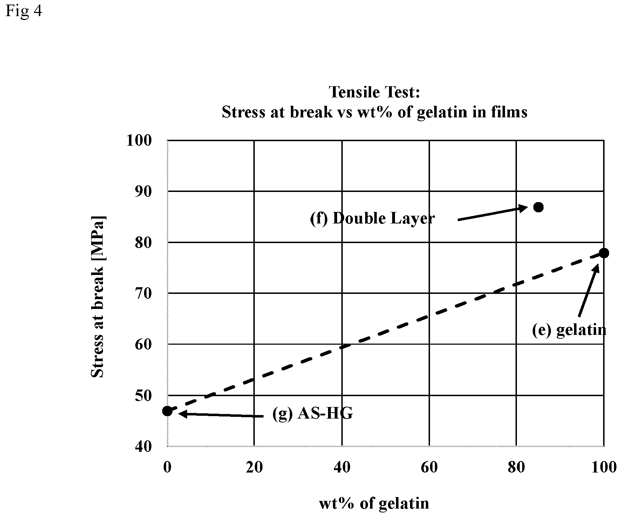

Figure 3 and Figure 4 show a graphical representation of the data of Table 26. The dashed line in Figure 3 and Figure 4 connecting the two extremes, AS-HG only and gelatin only, shows the expected values for the elastic modulus and for the stress at break in case of linear behavior. The Double Layer shows unexpected non-linear behavior.

The test results of the tensile test show unexpected non-linear improvements of the double layered films compared to films with similar thickness but with only one of the two polymers.

**Example 8: Dye penetration test**

Microtome cuts were prepared from two films:

- Double layered film HPMC-HPMCAS prepared according to Example 6.
- Double layered film gelatin-HPMCAS prepared according to Example 7.

In both cases the melt of the second layer was stained with 0.5 wt% pigment blue 2 (CAS 1325-94-6) based on weight of HPMCAS.

With a microscope, pictures under standard light were taken of these microtome cuts.

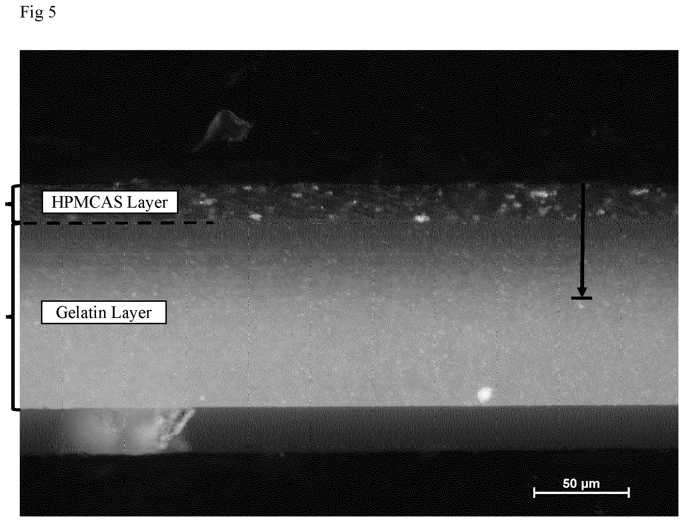

Figure 5 shows the picture of the microtome cut of the double layered film gelatin-HPMCAS.

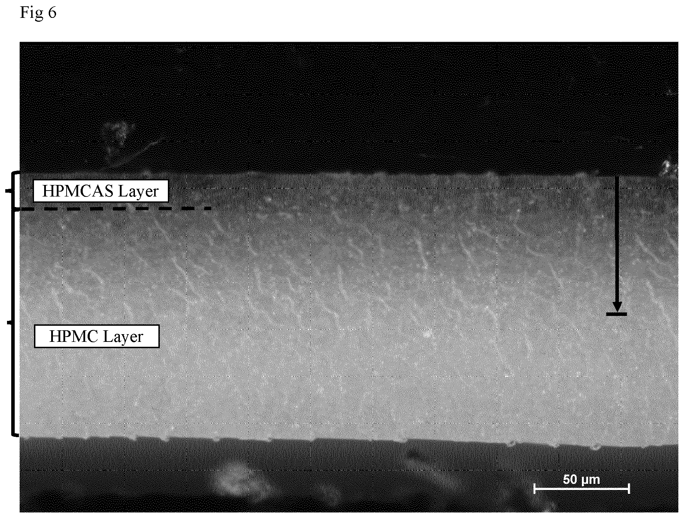

Figure 6 shows the picture of the microtome cut of the double layered film HPMC-HPMCAS. In the black and white version the penetration of the blue dye from the second, outer layer into the first, inner layer can bee seen, but more clearly it can be seen in the colored version of these pictures.

It is assumed that the combination of two aqueous based melts for MELT1 and MELT2 provides for this penetration of the dye from the second, that is from the outer layer into the first, that is into the inner layer, which distinguishes such double dipped capsules from coated capsules that are coated with an enteric polymer.

**NUMBERED STATEMENTS**

The invention further provides for the following:

Statement 1. A two piece hard capsule shell CAPSSHELL, wherein

CAPSSHELL is prepared by a double dipping process;
the wall of CAPSSHELL comprises two layers of wall forming polymers, one inner layer and one outer layer on the inner layer;
the wall forming polymer of the inner layer comprises a water soluble film forming polymer WATSOLPOL is selected from the group of gelatin, cellulose derivative, PVA, and modified starch;
the wall forming polymer of the outer layer comprises an delayed-release polymer ENTPOL selected from the group of HPMCAS, HPMCP, CAP, and polyacrylic acid copolymers.

Statement 2. The two piece hard capsule shell CAPSSHELL according to statement 1, wherein

WATSOLPOL is gelatin and
ENTPOL is selected from the group of HPMCAS, and polyacrylic acid copolymers; or
WATSOLPOL is selected from the group of cellulose derivative, PVA, and modified starch, and
ENTPOL is selected from the group of HPMCAS, HPMCP, CAP, and polyacrylic acid copolymers.

Statement 3. The two piece hard capsule shell CAPSSHELL according to statement 2, wherein
WATSOLPOL is gelatin or cellulose derivative.

Statement 4. The two piece hard capsule shell CAPSSHELL according to statement 2 or 3, wherein
WATSOLPOL is gelatin or HPMC.

Statement 5. The two piece hard capsule shell CAPSSHELL according to statement 4, wherein
the HPMC is selected from the group of:

HPMC 2910 containing about 7.0 to 12.0% hydroxypropyl group and about 28.0 to 30.0% methoxy group,
HPMC 2906 containing about 4.0 to 7.5% hydroxypropyl group and about 27.0 to 30.0% methoxy group,
HPMC 2208 containing about 4.0 to 12.0% of hydroxypropyl group and about 19.0 to 24.0% of methoxy group,
HPMC 1828 containing about 23.0 to 32.0% hydroxypropyl group and about 16.5 to 20.0% methoxy group, and
mixtures thereof.

Statement 6. The two piece hard capsule shell CAPSSHELL according to statement 4 or 5, wherein
the HPMC is HPMC 2906.

Statement 7. The two piece hard capsule shell CAPSSHELL according to one or more of statements 2 to 6, wherein
ENTPOL is HPMCAS, CAP or polyacrylic acid copolymers.

Statement 8. The two piece hard capsule shell CAPSSHELL according to one or more of statements 2 to 7, wherein
ENTPOL is HPMCAS or CAP.

Statement 9. The two piece hard capsule shell CAPSSHELL according to one or more of statements 2 to 8, wherein
ENTPOL is HPMCAS.

Statement 10. The two piece hard capsule shell CAPSSHELL according to one or more of statements 2 to 9, wherein
WATSOLPOL is cellulose derivative and ENTPOL is HPMCAS.

Statement 11. The two piece hard capsule shell CAPSSHELL according to one or more of statements 2, and 4 to
9, wherein
WATSOLPOL is HPMC and ENTPOL is HPMCAS.

Statement 12. The two piece hard capsule shell CAPSSHELL according to one or more of statements 2 to 4, and
7 to 9, wherein
WATSOLPOL is gelatin and ENTPOL is HPMCAS.

Statement 13. The two piece hard capsule shell CAPSSHELL according to one or more of statements 2 to 8, wherein
ENTPOL is CAP.

Statement 14. The two piece hard capsule shell CAPSSHELL according to one or more of statements 2, and 4 to
8, wherein
WATSOLPOL is gelatin and ENTPOL is CAP.

Statement 15. The two piece hard capsule shell CAPSSHELL according to one or more of statements 2 to 14, wherein
the amount of ENTPOL in CAPSSHELL may be at least 5 wt%, the wt% being based on dry capsule weight.

Statement 16. The two piece hard capsule shell CAPSSHELL according to one or more of statements 2 to 15, wherein
the amount of WATSOLPOL in CAPSSHELL is at least 50 wt%, the wt% being based on dry capsule weight.

Statement 17. The two piece hard capsule shell CAPSSHELL according to one or more of statements 2 to 16, wherein

the amount of WATSOLPOL in CAPSSHELL is from 67.5 to 90.5 wt%, and
the amount of ENTPOL in CAPSSHELL is from 9.5 to 32.5 wt%;
the wt% being based on dry capsule weight.

Statement 18. The two piece hard capsule shell CAPSSHELL according to one or more of statements 2 to 17, wherein

the inner or the outer layer or both layers of CAPSSHELL may further comprise a gelling system GELSYS;
GELSYS is one or more gelling agents GELAGE or a mixture of one or more GELAGE with one or more gelling aids GELAID;
GELAGE is selected from the group of hydrocolloid such as agar gum, guar gum, locust bean gum (carob), carrageenan, pectin, xanthan, gellan gum, konjac mannan, gelatin, and mixtures thereof;
GELAID is a cation; the cation may be $K^+$, $Na^+$, $Li^+$, $NH_4^+$, $Ca^{2+}$, $Mg^{2+}$ or mixtures thereof.

Statement 19. The two piece hard capsule shell CAPSSHELL according to one or more of statements 2 to 11, and 15 to 17, wherein

the inner layer does not comprise any GELSYS, and WATSOLPOL is HPMC;
with GELSYS as defined in statement 18.

Statement 20. The two piece hard capsule shell CAPSSHELL according to one or more of statements 2 to 19, wherein

CAPSSHELL comprises one or more further substances FURTHERSUBST,
FURTHERSUBST is selected from the group of plasticizer, pH regulator, sweetener,
acidulant, preservative, flavor, binder, thickener, colorant, and mixtures thereof.

Statement 21. The two piece hard capsule shell CAPSSHELL according to statement 20, wherein
the colorant is a dye or a pigment.

Statement 22. A method for preparation of CAPSSHELL by a dip molding process DOUBLEDIP,

in DOUBLEDIP a first dip molding DIP1 and a second dip molding DIP2 are done consecutively;
in DIP1 a first polymer film of WATSOLPOL is formed on a mold pin by dipping the mold pin into a first mixture MELT 1 and withdrawing it from the MELT1,
MELT1 comprises WATSOLPOL and water;
in DIP2 a second polymer film of ENTPOL is formed on the first polymer film on the mold pin by dipping the mold pin with the first polymer film from DIP1 into a second mixture MELT2 and withdrawing it from the MELT1,
MELT2 comprises ENTPOL and water;
with CAPSSHELL, WATSOLPOL and ENTPOL as defined in one or more of statements 1 to 21.

Statement 23. The method according to statement 22, wherein
MELT2 further comprises a base BAS2, BAS2 is ammonia.

Statement 24. The method according to statement 23, wherein
MELT2 comprises BAS2 in at least such an amount that ENTPOL is dissolved in MELT2.

Statement 25. The method according to one or more of statements 22 to 24, wherein DIP1 comprises the steps of:

(1-1) dipping the mold pin for one of the halves of CAPSSHELL into MELT1;
(1-2) withdrawing the mold pin from MELT1;
(1-3) allowing the first polymer film to form on the mold pin by drying.

Statement 26. The method according to one or more of statements 22 to 25, wherein DIP2 comprises the steps of:

(2-1) dipping the mold pin with the first polymer film from DIP1 into MELT2;
(2-2) withdrawing the mold pin from MELT2;
(2-3) allowing the second polymer film to form on the first polymer film on the mold pin by drying.

Statement 27. The method according to one or more of statements 22 to 26, wherein

a time DRYTIME1-2 is the time between the withdrawal of the mold pin from the MELT1 in DIP1 and the dipping of the mold pin into MELT2;
DRYTIME1-2 is not longer than 5 h.

Statement 28. A two piece hard capsule shell CAPSSHELL obtainable by DOUBLEDIP;

with CAPSSHELL as defined in one or more of statements 1 to 21;
with DOUBLEDIP as defined in one or more of statements 22 to 26.

Statement 29. A two piece hard capsule shell CAPSSHELL filled with a formulation FILLFORM, wherein

FILLFORM comprises an active ingredient ACTINGR, ACTINGR is selected from the group of active pharmaceutical ingredient, pharmaceutical dosage form, medicament, live biotherapeutic product, and nutritional product;
with CAPSSHELL as defined in one or more of statements 1 to 21 and 28.

Statement 30. The use of a two piece hard capsule shell CAPSSHELL as defined in one or more of statements 1 to 21 and 28 for filling with FILLFORM comprising an active ingredient ACTINGR,
with FILFORM and ACTINGR as defined in statement 29.

Statement 31. The use of a two piece hard capsule shell CAPSSHELL as defined in one or more of statements 1 to 21 and 28 filled with FILLFORM for oral intake; FILLFORM comprising an active ingredient ACTINGR,
with FILFORM and ACTINGR as defined in statement 29.

**Claims**

1. A two piece hard capsule shell CAPSSHELL, wherein

   CAPSSHELL is prepared by a double dipping process;
   the wall of CAPSSHELL comprises two layers of wall forming polymers, one inner layer and one outer layer on the inner layer;
   the wall forming polymer of the inner layer comprises a water soluble film forming polymer WATSOLPOL being gelatin;
   the wall forming polymer of the outer layer comprises an delayed-release polymer ENTPOL being CAP.

2. The two piece hard capsule shell CAPSSHELL according to one or more of claim 1 to 3, wherein
   the amount of ENTPOL in CAPSSHELL may be at least 5 wt%, the wt% being based on dry capsule weight.

3. The two piece hard capsule shell CAPSSHELL according to claim 1 or 2, wherein
   the amount of WATSOLPOL in CAPSSHELL is at least 50 wt%, such as from 50 to 95 wt%, preferably from 55 to 92.5 wt%, more preferably from 60 to 90 wt%, even more preferably from 65 to 90 wt%, especially from 67.5 to 90.5 wt%, the wt% being based on dry capsule weight.

4. The two piece hard capsule shell CAPSSHELL according to one or more of claims 1 to 3, wherein
   the amount of ENTPOL in CAPSSHELL is from 5 to 50 wt%, preferably from 7.5 to 45 wt%, more preferably from 7.5 to 40 wt%, even more preferably from 7.5 to 35 wt%, especially from 9.5 to 35 wt%, more especially from 9.5 to 32.5 wt%, the wt% being based on dry capsule weight.

5. The two piece hard capsule shell CAPSSHELL according to one or more of claims 1 to 4, wherein
   the amount of WATSOLPOL in CAPSSHELL is from 77.5 to 90.5 wt%, the wt% being based on dry capsule weight.

6. The two piece hard capsule shell CAPSSHELL according to one or more of claims 1 to 5, wherein

   the amount of WATSOLPOL in CAPSSHELL is from 77.5 to 90.5 wt%, and
   the amount of ENTPOL in CAPSSHELL is from 9.5 to 22.5 wt%;
   the wt% being based on dry capsule weight.

**7.** The two piece hard capsule shell CAPSSHELL according to one or more of claims 1 to 6, wherein

the inner or the outer layer or both layers of CAPSSHELL may further comprise a gelling system GELSYS;
GELSYS is one or more gelling agents GELAGE or a mixture of one or more GELAGE with one or more gelling aids GELAID;
GELAGE is selected from the group of hydrocolloid such as agar gum, guar gum, locust bean gum (carob), carrageenan, pectin, xanthan, gellan gum, konjac mannan, gelatin, and mixtures thereof;
GELAID is a cation; the cation may be $K^+$, $Na^+$, $Li^+$, $NH_4^+$, $Ca^{2+}$, $Mg^{2+}$ or mixtures thereof.

**8.** The two piece hard capsule shell CAPSSHELL according to one or more of claims 1 to 7, wherein

the inner layer does not comprise any GELSYS;
with GELSYS as defined in claim 8.

**9.** The two piece hard capsule shell CAPSSHELL according to one or more of claims 1 to 8, wherein

CAPSSHELL comprises one or more further substances FURTHERSUBST,
FURTHERSUBST is selected from the group of plasticizer, pH regulator, sweetener, acidulant, preservative, flavor, binder, thickener, colorant, and mixtures thereof.

**10.** The two piece hard capsule shell CAPSSHELL according to claim 9, wherein
the colorant is a dye or a pigment.

**11.** A method for preparation of CAPSSHELL by a dip molding process DOUBLEDIP,

in DOUBLEDIP a first dip molding DIP1 and a second dip molding DIP2 are done consecutively;
in DIP1 a first polymer film of WATSOLPOL being gelatin is formed on a mold pin by dipping the mold pin into a first mixture MELT 1 and withdrawing it from the MELT 1,
MELT1 comprises WATSOLPOL and water;
in DIP2 a second polymer film of ENTPOL being HPMCAS is formed on the first polymer film on the mold pin by dipping the mold pin with the first polymer film from DIP1 into a second mixture MELT2 and withdrawing it from the MELT1,
MELT2 comprises ENTPOL and water;
with CAPSSHELL, WATSOLPOL and ENTPOL as defined in one or more of claims 1 to 10.

**12.** The method according to claim 11, wherein
MELT2 further comprises a base BAS2, BAS2 is ammonia.

**13.** The method according to claim 12, wherein
MELT2 comprises BAS2 in at least such an amount that ENTPOL is dissolved in MELT2.

**14.** The method according to one or more of claims 11 to 13, wherein
DIP1 comprises the steps of:

(1-1) dipping the mold pin for one of the halves of CAPSSHELL into MELT1;
(1-2) withdrawing the mold pin from MELT1;
(1-3) allowing the first polymer film to form on the mold pin by drying.

**15.** The method according to one or more of claims 11 to 14, wherein
DIP2 comprises the steps of:

(2-1) dipping the mold pin with the first polymer film from DIP1 into MELT2;
(2-2) withdrawing the mold pin from MELT2;
(2-3) allowing the second polymer film to form on the first polymer film on the mold pin by drying.

**16.** The method according to one or more of claims 11 to 15, wherein

a time DRYTIME1-2 is the time between the withdrawal of the mold pin from the MELT1 in DIP1 and the dipping of the mold pin into MELT2;
DRYTIME1-2 is not longer than 5 h.

17. A two piece hard capsule shell CAPSSHELL obtainable by DOUBLEDIP;

with CAPSSHELL as defined in one or more of claims 1 to 10;
with DOUBLEDIP as defined in one or more of claims 11 to 16.

18. A two piece hard capsule shell CAPSSHELL filled with a formulation FILLFORM, wherein

FILLFORM comprises an active ingredient ACTINGR, ACTINGR is selected from the group of active pharmaceutical ingredient, pharmaceutical dosage form, medicament, live biotherapeutic product, and nutritional product;
with CAPSSHELL as defined in one or more of claims 1 to 10 and 17.

19. The use of a two piece hard capsule shell CAPSSHELL as defined in one or more of claims 1 to 11 and 18 for filling with FILLFORM comprising an active ingredient ACTINGR,
with FILFORM and ACTINGR as defined in claim 18.

20. The use of a two piece hard capsule shell CAPSSHELL as defined in one or more of claims 1 to 11 and 18 filled with FILLFORM for oral intake; FILLFORM comprising an active ingredient ACTINGR,
with FILFORM and ACTINGR as defined in claim 18.

Fig 1

**Puncture Test:**
**Force at break vs wt% of HPMC in films**

**wt% of HPMC**

Fig 2

**Tensile Test:**
**Deformation at break vs wt% of HPMC in films**

Fig 3

**Tensile Test:**
**Elastic modulus vs wt% of gelatin in films**

Fig 4

**Tensile Test:
Stress at break vs wt% of gelatin in films**

Fig 5

Fig 6

## REFERENCES CITED IN THE DESCRIPTION

**Patent documents cited in the description**

- GB 2361643 A
- GB 1455884 A
- US 4816259 A
- US 20130295188 A1

**Non-patent literature cited in the description**

- *CHEMICAL ABSTRACTS,* 9004-65-3
- Pharmaceutical Capsules. Pharmaceutical Press, 2014, 84
- *CHEMICAL ABSTRACTS,* 103-90-2
- *CHEMICAL ABSTRACTS,* 1325-94-6